# FASCICULE DE BREVET EUROPEEN

(11) **EP 2 491 008 B1**
(45) Date de publication et mention de la délivrance du brevet: **21.08.2019**
(21) Numéro de dépôt: 10782662.0
(22) Date de dépôt: 18.10.2010
(51) Int. Cl.: C07C 235/06, A01N 25/02

(54) **COMPOSES DE TYPE ETHER-AMIDE ET UTILISATIONS**
ETHER-AMID-VERBINDUNGEN UND IHRE VERWENDUNG
ETHER-AMIDE COMPOUNDS AND USES THEREOF

(30) Priorité: 19.10.2009 FR 0905000
(43) Date de publication de la demande: 29.08.2012
(73) Titulaire: Rhodia Operations, 93306 Aubervilliers (FR)
(72) Inventeur: VIDAL, Thierry, 69003 Lyon (FR); GUGLIERI, Massimo, 98000 Monaco (MC); JENTZER, Olivier, 69390 Vourles (FR)
(74) Mandataire: Cardon, Flavie
(86) Numéro de dépôt international: PCT/FR2010/052212
(87) Numéro de publication internationale: WO 2011/048314

(56) Documents cités:
- WO-A1-2006/003210
- WO-A2-2007/107745
- CH-A- 349 440
- CH-A5- 573 702
- DE-A1- 4 341 986
- FR-A- 922 954
- FR-A1- 2 564 288
- GB-A- 579 887
- US-A- 2 447 587
- US-A- 4 379 928
- US-A- 4 775 673
- US-A- 5 693 126
- US-A- 6 136 822
- ANNUNZIATA R ET AL: "CHELATION AND NON-CHELATION CONTROLLED STEREOSELECTIVE REDUCTION OFALPHA-METHOXY-ALPHA-PHENYLTHIO KETONES" TETRAHEDRON, vol. 47, no. 23, 1 janvier 1991 (1991-01-01), pages 3853-3868, XP002027018 ELSEVIER SCIENCE PUBLISHERS, AMSTERDAM, NL ISSN: 0040-4020 DOI: 10.1016/S0040-4020(01)80909-1
- MAKOTO FUJITA: "Flouride ion Catalyzed Reduction of Aldehydes and Ketones with Hydrosilanes. Synthetic and Mechanistic Aspects and Application to the Threo-Directed Reduction of alpha-Substituted Alkanones" JOURNAL OF ORGANIC CHEMISTRY, vol. 53, no. 23, 1 janvier 1988 (1988-01-01), pages 5405-5415, XP002172516 AMERICAN CHEMICAL SOCIETY, EASTON.; US ISSN: 0022-3263 DOI: 10.1021/JO00258A003
- ERACH R. TALATY: "Regioselectivity in nucleophilic ring-opening of aziridinones" CHEMICAL COMMUNICATIONS, 31 décembre 1998 (1998-12-31), pages 985-986, XP002584736 ISSN: 1359-7345
- PELLISIER: "REACTIONS OF ISOCYANIDES. II - ADDITION TO ACETALS" TETTRAHEDRON LETTERS, vol. 27, no. 30, 31 décembre 1986 (1986-12-31), pages 3505-3506, XP002584737 ELSEVIER, AMSTERDAM, NL ISSN: 0040-4039
- KOBAYASHI Y. ET AL: "An Improved Synthetic Method of (S)-2-Alkoxypropanals from Ethyl (S)-Lactate" BULLETIN OF THE CHEMICAL SOCIETY OF JAPAN, vol. 62, no. 9, septembre 1989 (1989-09), pages 3038-3040, XP002623249 ISSN: 0009-2673 DOI: 10.1246/bcsj.62.3038
- WRIGHT J. B. ET AL: "Analogs of Amphenone. The Synthesis of Aminosubstituted Diphenylacetones and Related Compounds" JOURNAL OF THE AMERICAN CHEMICAL SOCIETY, vol. 81, no. 19, 5 octobre 1959 (1959-10-05), pages 5193-5199, XP002623250 DOI: 10.1021/ja01528a043
- RAMÍREZ A. ET AL: "Hemilabile Ligands in Organolithium Chemistry: Substituent Effects on Lithium Ion Chelation" JOURNAL OF THE AMERICAN CHEMICAL SOCIETY, vol. 125, no. 50, 17 décembre 2003 (2003-12-17), pages 15376-15387, XP002623251 DOI: 10.1021/ja030322d -& RAMÍREZ A. ET AL: "Supporting Information: Hemilabile Ligands in Organolithium Chemistry: Substituent Effects on Lithium Ion Chelation" JOURNAL OF THE AMERICAN CHEMICAL SOCIETY, [Online] 17 décembre 2003 (2003-12-17), pages 41-58, XP002627203 American Chemical Society Extrait de l'Internet: URL:http://pubs.acs.org/doi/suppl/10.1021/ ja030322d/suppl_file/ja030322d_sa.pdf> [extrait le 2011-02-17]
- DE VOGHEL ET AL: "Phosgene Immonium salts" JOURNAL OF ORGANIC CHEMISTRY, vol. 39, no. 9, 1 janvier 1974 (1974-01-01), pages 1233-1235, XP002157319 AMERICAN CHEMICAL SOCIETY, EASTON.; US ISSN: 0022-3263 DOI: 10.1021/JO00923A015
- SPEZIALE A. J. ET AL: "Synthesis of Cyclopropanetricarboxamides" JOURNAL OF ORGANIC CHEMISTRY, vol. 30, no. 4, avril 1965 (1965-04), pages 1199-1202, XP002623252 DOI: 10.1021/jo01015a057
- CHUNG S. J. ET AL: "Convenient synthesis of 6-substituted-2-chloro-5,12-dihydro-5-oxob enzoxazolo[3,2-a]quinolines and N-acylated-3-chlorodibenz[b,e][1,4]oxazepi n-11(5H)-ones" JOURNAL OF HETEROCYCLIC CHEMISTRY (MARCH/APRIL 1997), vol. 34, no. 2, mars 1997 (1997-03), pages 485-488, XP002623253 DOI: 10.1002/jhet.5570340221
- BOROWITZ I J ET AL: "Convenient One-Step Conversions of Alcohols orPhenols to N,N-Dipropyl Alkoxyacetamides" ORGANIC PREPARATIONS AND PROCEDURES INTERNATIONAL, ORGANIC PREPARATION AND PROCEDURES CO., NEWTON HIGHLANDS, MA, US, vol. 9, no. 6, 1 janvier 1977 (1977-01-01), pages 257-262, XP009144618 ISSN: 0030-4948

## Description

La présente invention a pour objet de nouveaux composés, de type éther-amide. Elle a également pour objet des utilisations de tels composés, notamment comme solvants, par exemple dans des formulations phytosanitaires.

L'industrie utilise de nombreux composés chimiques à titre de solvants, par exemple pour préparer des produits chimiques et des matériaux, pour formuler des composés chimiques, ou pour traiter des surfaces. Par exemple, des solvants sont utilisés pour la formulation d'actifs phytosanitaires notamment sous forme de concentrés émulsionnables (Emulsifiable Concentrate "EC") destinés à être dilués dans de l'eau par l'exploitant agricole, avant application sur un champ. Des solvants sont aussi utilisés pour la formulation d'actifs phytosanitaires notamment sous forme de microémulsions ("ME") ou d'émulsions dans l'eau (Emulsion in Water "EW") destinées à être dilués dans de l'eau par l'exploitant agricole, avant application sur un champ.

L'industrie est à la recherche de nouveaux composés permettant de varier ou d'optimiser les produits et procédés dans lesquels des solvants, notamment des solvants polaires, sont à utiliser. L'industrie a notamment besoin de composés de coût modeste, présentant des propriétés d'usage intéressantes. L'industrie a également besoin de composés présentant un profil toxicologique et/ou écologique perçu comme favorable, notamment une faible volatilité (faible VOC), une bonne biodégradabilité, une faible toxicité et/ou une faible dangerosité.

On connaît l'utilisation comme solvants des diméthylamides. Il s'agit de produits de formule R-CONMe₂ où R est un groupe hydrocarboné comme un alkyle, typiquement en C₆-C₃₀. De tels produits sont notamment commercialisés sous la dénomination Genagen® par la société Clariant. Ces solvants trouvent des applications notamment dans le domaine phytosanitaire. Ces solvants présentent toutefois un domaine d'utilisation restreint et ne permettent pas de solubiliser certains actifs phytosanitaires à certaines concentrations, dans des gammes de températures utiles, sans formation de cristaux.

Les documents WO 2007/107745, WO 2009/027624 et WO 2009/027626 (Syngenta) décrivent l'utilisation comme solvant de lactamides (alpha-hydroxyamides), notamment dans le domaine phytosanitaire. Il existe un besoin pour d'autres solvants.

Le document WO 2008/101629 (Cognis) décrit l'utilisation comme solvant de lactamides (alpha-hydroxyamides), notamment dans le domaine phytosanitaire. Il existe un besoin pour d'autres solvants.

Le document GB 579 887 décrit des solutions de polymère d'acrylonitrile contenant au moins 85% en poids d'acrylonitrile dans un ou plusieurs solvants volatiles. Il décrit notamment à titre de solvant, le diméthyl méthoxyacétamide de formule MeO-CH₂-CONMe₂.

Le document DE 43 41 986 décrit l'utilisation de composés R¹-CO-NR²R³ comme inhibiteurs de cristallisation, ces composés étant des composés de type linéaires.

Il existe aussi un besoin pour des plastifiants.

Par ailleurs le document US 3827994 (Grace) décrit des composés utiles dans des compostions de résines aminoplastes pour traiter les textiles ou papiers. Les composés sont dits améliorer des propriétés comme la résistance au froissage des textiles. Le composé de formules CH₃-CH(OMe)-CO-NMe₂ est notamment divulgué en colonne 26. D'autres propriétés ou usages ne sont pas suggérés.

Il demeure un besoin pour de nouveaux solvants, co-solvants, inhibiteurs de cristallisation, ou agents plastifiants, notamment dans les formulations phytosanitaires, et pour de nouveaux composés, pouvant notamment élargir la gamme d'actifs pouvant être formulés et/ou leur concentration et/ou pouvant élargir les conditions d'utilisation, notamment en terme de stabilité par exemple sans formation de cristaux, à basse température.

La présente invention répond à au moins un des besoins exprimés ci-dessus en proposant l'utilisation comme solvant, co-solvant, inhibiteur de cristallisation, ou agent plastifiant d'un composé de formule (I) suivante:

R^{a}R^{b}C(OR¹)-CONR²R³ (I)

où
- R^{a} est un groupe alkyle, linéaire ou ramifié, en C₁-C₆,
- R^{b} est H,
- R¹ est choisi parmi les groupes méthyle, éthyle, propyle, isopropyle, n-butyle, isobutyle, n-pentyle, isopentyle, isoamyle, n-hexyle, cyclohexyle, n-octyle, isooctyle, 2-éthylhexyle, décyle, dodécyle, tridécyle, phényle et 1-phényléthyle,
- R² et R³, identiques ou différents, sont des groupes hydrocarbonés comprenant un nombre moyen d'atomes de carbone allant de 1 à 15, éventuellement substitués, choisi parmi les groupes aliphatiques acycliques, saturés ou insaturés, linéaires ou ramifiés, les groupes carbocycliques saturés, insaturés ou aromatiques, monocycliques ou polycycliques et les enchaînements desdits groupes,
   R² et R³ pouvant éventuellement former ensemble un cycle comprenant l'atome d'azote auquel ils sont liés, éventuellement substitué.

La présente demande décrit également l'utilisation comme solvant, co-solvant, inhibiteur de cristallisation, ou agent plastifiant d'un composé de formule (I) suivante:

R^{a}R^{b}C(OR¹)-CONR²R³ (I)

où
- R^{a} et R^{b}, identiques ou différents, sont des groupes choisis parmi l'atome d'hydrogène et les groupes alkyles linéaires ou ramifiés, de préférence en C₁-C₆, de préférence en C₁-C₃,
- R¹ est un groupe R'¹ ou -(AO)ₙR'¹, où
   - R'¹ est un groupe choisi parmi les groupes hydrocarbonés comprenant un nombre moyen d'atomes de carbone allant de 1 à 36, de préférence de 1 à 15, saturés ou insaturés, linéaires ou ramifiés, éventuellement cycliques, éventuellement aromatiques lesdits groupes aromatiques pouvant comprendre un hétéroatome dans un cycle aromatique,
   - AO, identique ou différent, représente un groupe de formule -CH₂-CH₂-O-,
   - CHMe-CH₂-O-, ou -CH₂-CHMe-O-
   - n est un nombre moyen supérieur ou égal à 0, allant par exemple de 0 à 50,
- R² et R³, identiques ou différents, sont des groupes hydrocarbonés comprenant un nombre moyen d'atomes de carbone allant de 1 à 36, de préférence de 1 à 15, saturés ou insaturés, linéaires ou ramifiés, éventuellement cycliques, éventuellement aromatiques, éventuellement substitués, R² et R³ pouvant éventuellement former ensemble un cycle comprenant l'atome d'azote auquel ils sont liés, éventuellement substitué et/ou comprenant éventuellement un hétéroatome supplémentaire,
   avec la condition que si R^{a}=R^{b}=H, le composé de formule (I) est utilisé dans une formulation phytosanitaire.

La présente invention a également pour objet de nouveaux composés, pouvant être particulièrement appropriés à des utilisations, à des procédés ou à la mise en oeuvre de formulations mentionnées plus haut.

### Définitions ou abréviations

Dans la présente demande on utilise notamment les abréviations suivantes: Me signifie méthyle; Et signifie éthyle; IsoAm signifie Isoamyle, cyclo ou Cy signifie cyclohexyle.

Dans la présente demande on entend par "composé de l'invention" un composé de formule (I).

Dans la présente demande on entend par "composés nouveaux de l'invention" certains composés de formule (I).

Dans la présente demande, par "composition de matière", on entend une composition, plus ou moins complexe, comprenant plusieurs composés chimiques. Il peut s'agir typiquement d'un produit de réaction non purifié ou modestement purifié. Le composé de l'invention pourra notamment être isolé et/ou commercialisé et/ou utilisé sous forme d'une composition de matière le comprenant. Le composé de l'invention, sous forme de molécule pure ou sous forme d'un mélange répondant à la formule (I), peut être compris dans une composition de matière.

Dans la présente demande le terme solvant est entendu dans un sens large, couvrant notamment les fonctions de co-solvant, d'inhibiteur de cristallisation, de décapant. Le terme solvant peut notamment désigner un produit liquide à la température d'utilisation, de préférence de point de fusion inférieur ou égal à 20°C, de préférence à 5°C, de préférence à 0°C, pouvant contribuer à rendre liquide une matière solide, ou à empêcher ou retarder la solidification ou la cristallisation de matière dans un milieu liquide.

Dans la présente demande, l'expression "groupes hydrocarbonés" désigne notamment des groupes aliphatiques acycliques, saturés ou insaturés, linéaires ou ramifiés, ou des groupes carbocycliques ou hétérocycliques saturés, insaturés ou aromatiques, monocycliques ou polycycliques, ou encore des enchaînements desdits groupes.

### Composé de l'invention

Le composé de l'invention présente la formule générale (I) donnée ci-dessus. On note qu'il peut s'agir d'un mélange de plusieurs composés de formule générale (I). En d'autres termes le composé peut être seul ou en mélange. Dans le cadre de mélanges de plusieurs composés les nombres d'atomes ou de motifs peuvent être exprimés en nombres moyens. Il s'agit de nombres moyens en nombre. Dans le cas de composés seuls, il s'agira généralement de nombres entiers, en ce qui concerne le nombre d'atomes de carbone.

On mentionne que R^{a} est un groupe choisi parmi les groupes alkyles linéaires ou ramifiés. Les alkyles sont des alkyles en C₁-C₆, de préférence en C₁-C₄ et préférentiellement en C₁-C₃. Il peut notamment s'agir de groupes méthyle, éthyle, propyle ou butyle. Selon un mode de réalisation particulier, le nombre total d'atomes de carbone, hors groupes R¹, R² et R³ est de 3, 4, 5 ou 6 ou des nombres moyens entre chacune de ces valeurs.

Le mode de réalisation où R^{a}=Me, et R^{b}=H peut correspondre à des composés dérivés de l'acide lactique de formule CH₃-CHOH-COOH avec un éther - OR¹ à la place l'hydroxyle et une amide -CONR²R³ à la place de l'acide -COOH.

Selon l'invention, les composés de formule (I) sont caractérisés en ce que R^{a} est un groupe alkyle, linéaire ou ramifié, en C₁-C₆, et R^{b} est H De tels composés sont également appelés composés ramifiés'.

Selon un mode de réalisation particulier "G" pour le composé de l'invention, R^{a} = Me, R^{b} = H et R¹ est un groupe différent du groupe méthyle. Tout ce qui suit dans ce paragraphe concerne ce mode de réalisation particulier "G". Selon un mode de réalisation particulier R^{a} = Me, R^{b} = H et R¹ est un groupe différent du n-butyle. Selon un mode de réalisation particulier R^{a} = Me, R^{b} = H et R¹ est un groupe alkyle comprenant de 2 à 5 atomes de carbone, à l'exception du n-butyle, par exemple un groupe éthyle, n-propyle, isopropyle, isobutyle, tertbutyle, n-pentyle, isopentyle, isoamyle. Selon un mode de réalisation particulier R^{a} = Me, R^{b} = H et R¹ est un groupe cyclique, de préférence un groupe cyclohexyle. Selon un mode de réalisation particulier R¹ est un groupe différent groupe méthyle. Selon un mode de réalisation particulier R¹ est un groupe différent du n-butyle. Selon un mode de réalisation particulier R¹ est un groupe cyclique, de préférence un groupe cyclohexyle. Selon un mode de réalisation particulier R¹ est un groupe alkyle comprenant de 2 à 5 atomes de carbone, à l'exception du n-butyle, par exemple un groupe éthyle, n-propyle, isopropyle, isobutyle, tertbutyle, n-pentyle, isopentyle, isoamyle. Selon un mode de réalisation particulier R^{a} = Me, R^{b} = H et si R¹ est un groupe alkyle, alors il comprend plus de 5 atomes de carbone, R¹ pouvant par exemple être un groupe n-hexyle, cyclohexyle, n-octyle, isooctyle, 2-éthylhexyle, décyle, dodécyle, tridécyle, phényle ou benzyle. Selon un mode de réalisation particulier R^{a} = Me, R^{b} = H et R¹ est un groupe alkyle, comprenant plus de 5 atomes de carbone.

La présente demande décrit également l'utilisation comme solvant, co-solvant, inhibiteur de cristallisation, ou agent plastifiant, dans une formulation phytosanitaire, d'un composé de formule (I-1) suivante :

R¹O-H₂C-CONR²R³ (I-1)

où
- R¹ est un groupe R'¹ ou -(AO)ₙR'¹, où
   - R'¹ est un groupe choisi parmi les groupes hydrocarbonés comprenant un nombre moyen d'atomes de carbone allant de 1 à 15, éventuellement substitués, choisis parmi les groupes aliphatiques acycliques, saturés ou insaturés, linéaires ou ramifiés, les groupes carbocycliques ou hétérocycliques saturés, insaturés ou aromatiques, monocycliques ou polycycliques, et les enchaînements desdits groupes,
   - AO, identique ou différent, représente un groupe de formule -CH₂-CH₂-O-,
   - CHMe-CH₂-O-, ou -CH₂-CHMe-O-
   - n est un nombre moyen supérieur ou égal à 0,
- R² et R³, identiques ou différents, sont des groupes hydrocarbonés comprenant un nombre moyen d'atomes de carbone allant de 1 à 15, éventuellement substitués, choisi parmi les groupes aliphatiques acycliques, saturés ou insaturés, linéaires ou ramifiés, les groupes carbocycliques ou hétérocycliques saturés, insaturés ou aromatiques, monocycliques ou polycycliques et les enchaînements desdits groupes,
   R² et R³ pouvant éventuellement former ensemble un cycle comprenant l'atome d'azote auquel ils sont liés, éventuellement substitué et/ou comprenant éventuellement un hétéroatome supplémentaire.

Dans la formule (I), les groupes R² et R³, identiques ou différents, sont des groupes choisis parmi l'atome d'hydrogène et les groupes hydrocarbonés comprenant un nombre moyen d'atomes de carbone allant de 1 à 36, saturés ou insaturés, linéaires ou ramifiés, éventuellement cycliques, éventuellement aromatiques, éventuellement substitués, R² et R³ pouvant éventuellement former ensemble un cycle comprenant l'atome d'azote auquel ils sont liés, éventuellement substitué et/ou comprenant éventuellement un hétéroatome supplémentaire. Il est à noter que R² et R³ ne sont pas simultanément des atomes d'hydrogène. En d'autres termes le groupe -CONR²R³ n'est pas un groupe -CONH₂. Il peut s'agir d'un groupe -CONHR² où R² n'est pas un atome d'hydrogène, ou d'un groupe -CONR²R³ où R² et R³ ne sont pas des atomes d'hydrogène. R² et R³, identiques ou différents, peuvent par exemple être choisis parmi les groupes méthyle, éthyle, propyle (n-propyl), isopropyle, n-butyle, isobutyle, n-pentyle, amyle, isoamyle, hexyle, cyclohexyle, leurs mélanges et/ou associations. R² et R³ peuvent également être tels qu'ils forment ensemble avec l'atome d'azote un groupe morpholine, pyrrolidine, pipérazine ou pipéridine. R² et R³ peuvent notamment être des groupes méthyle, de préférence tous les deux.

Le groupe R¹ est un groupe typiquement correspondant à un alcool R¹-OH. Dans un cas il peut correspondre à un alcool simple R'¹-OH. Dans un autre cas il peut correspondre à un alcool éthoxylé et/ou propoxylé de formule HO-(AO)ₙR'¹. R'¹ représente le reste hydrocarboné de ces alcools éventuellement éthoxylés et/ou propoxylés. Le groupe R'¹ est un groupe choisi parmi les groupes hydrocarbonés comprenant un nombre moyen d'atomes de carbone allant de 1 à 36, saturés ou insaturés, linéaires ou ramifiés, éventuellement cycliques, éventuellement aromatiques lesdits groupes aromatiques pouvant comprendre un hétéroatome dans un cycle aromatique. L'hétéroatome du groupe aromatique peut être un atome d'oxygène ou d'azote. On mentionne que le groupe aromatique peut être directement relié ou être porté par un groupe alkyle. On mentionne que les groupes cycliques ou aromatiques peuvent être substitués. R'¹ peut par exemple être choisi parmi les groupes méthyle, éthyle, propyle, isopropyle, n-butyle, isobutyle, n-pentyle, isopentyle, isoamyle, n-hexyle, cyclohexyle, n-octyle, isooctyle, 2-éthylhexyle, décyle, dodécyle, tridécyle, phényle, benzyle et leurs mélanges. On note que R'¹ peut être un mélange plus ou moins complexe, pouvant correspondre à la mise en oeuvre d'un mélange plus ou moins complexe d'alcools R¹-OH, par exemple de l'huile de fusel.

Selon un mode de réalisation particulier le groupe R¹ est un groupe cyclique, de préférence un groupe cyclohexyle.

Le groupe (AO) représente un groupe éthoxy de formule -CH₂-CH₂-O- ou un groupe propoxy de formule -CHMe-CH₂-O-, ou -CH₂-CHMe-O-. Le nombre n est un nombre moyen supérieur ou égal à 0, allant par exemple de 0 à 50. Il représente typiquement un degré d'éthoxylation et/ou propoxylation. Dans le cas où des groupes éthoxy et propoxy sont présents, ils peuvent être distribués de manière aléatoire ou de manière séquencée.

Le composé de l'invention est de préférence tel qu'il présente un point de fusion inférieur à ou égal à 20°C, de préférence à 5°C, de préférence à 0°C. Les groupes détaillés ci-dessus sont de préférence tels que le composé présente cette propriété.

Selon un mode de réalisation le composé de l'invention peut être totalement miscible dans l'eau. Selon un mode de réalisation particulier, le composé de l'invention est partiellement miscible dans l'eau. La miscibilité dans l'eau peut par exemple être inférieure à 10% en poids (à 25°C), de préférence à 2%, de préférence à 1% ou à 0,1%. Elle peut être supérieure à 0,001%, de préférence à 0,01% ou à 0,1%. Elle peut par exemple être comprise entre 0,01% et 2%, par exemple entre 0,1% et 1%. De manière surprenante les composés de l'invention présentent de bonnes propriétés solvantes, notamment pour des actifs phytosanitaires dans des formulations phytosanitaires avec une faible miscibilité dans l'eau. Les groupes R^{a}, R^{b}, et/ou le groupe R¹ et/ou les groupes R², R³ peuvent être choisis de manière à contrôler la miscibilité dans l'eau.

Le composé de l'invention peut notamment présenter l'une des formules suivantes :

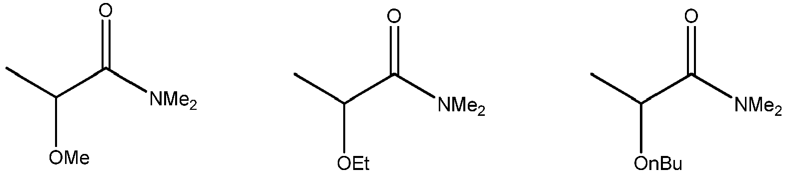

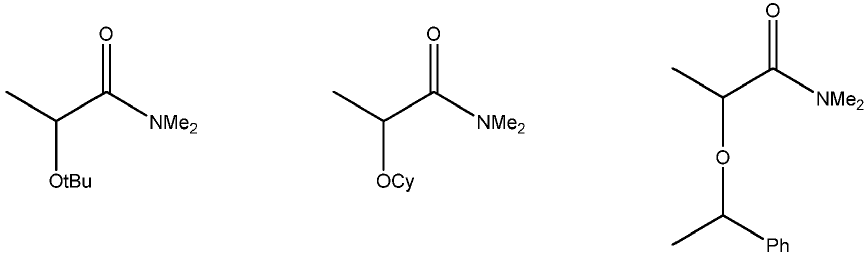

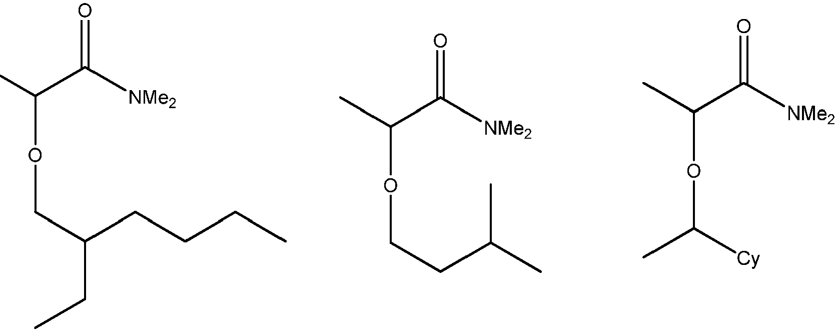

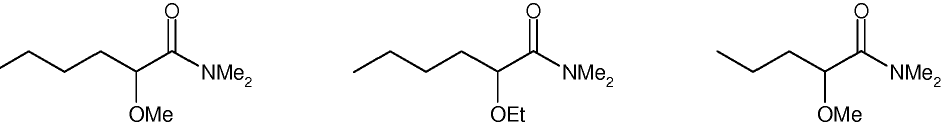

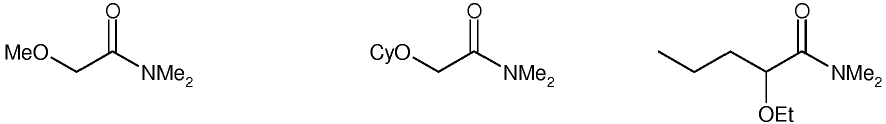

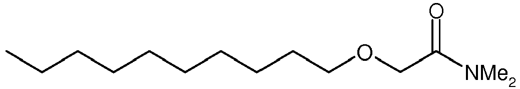

Le composé de l'invention peut notamment être un des composés nouveaux de l'invention, détaillés plus bas.

On mentionne que le composé de l'invention peut être compris dans une composition de matière, comprenant d'autres produits que le composé seul ou en mélange répondant à la formule (I). Dans la composition de matière, le composé de l'invention peut représenter au moins 10% en poids. De préférence, il s'agit du composé principal de la composition de matière. Par composé principal, on entend dans la présente demande, le composé dont la teneur est la plus élevée, même si sa teneur est inférieure à 50% en poids (par exemple dans un mélange de 40% de A, de 30% de B, et de 30% de C, le produit A est le composé principal). Encore plus préférablement, le composé de l'invention représente au moins 50% en poids de la composition de matière, par exemple de 70 à 95% en poids, et même de 75 à 90% en poids. Comme indiqué plus haut, la composition de matière peut être un produit de réaction. Les autres produits de la composition de matière peuvent notamment être des sous-produits des impuretés, des produits n'ayant pas réagi, ou des produits correspondant à des adduits de réaction de produits compris dans les composés de départ ne menant pas aux composés de formule (I).

### Composés nouveaux de l'invention

L'invention concerne également certains composés nouveaux de formule (I). Ces composés nouveaux, particuliers, peuvent être mis en oeuvre à titre de composés de l'invention.

Parmi les composés ramifiés préférés, on peut notamment citer les composés suivants :

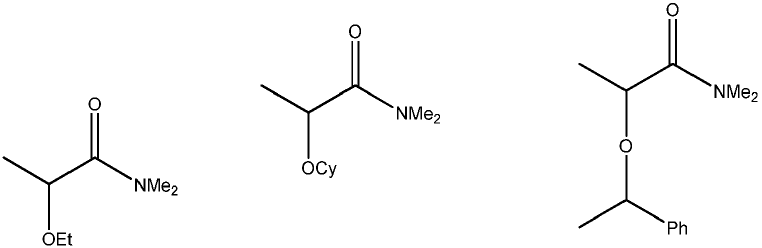

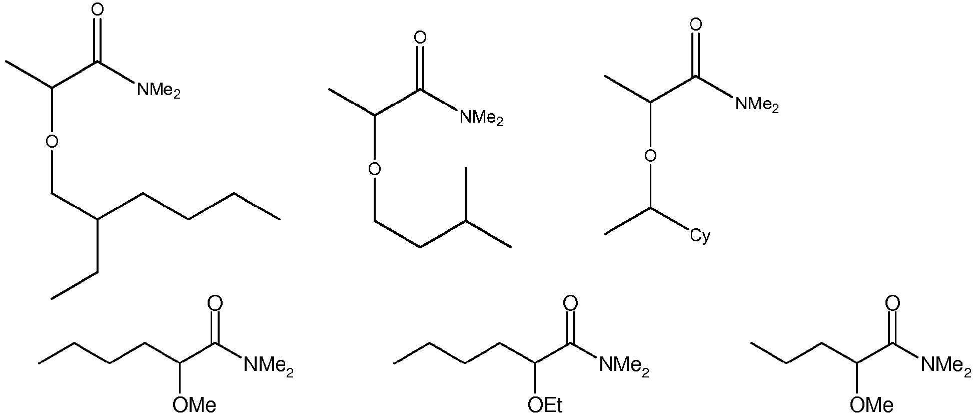

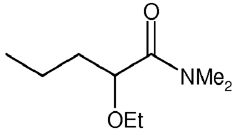

Les composés nouveaux de l'invention peuvent notamment être choisis parmi les composés suivants :

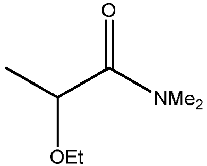

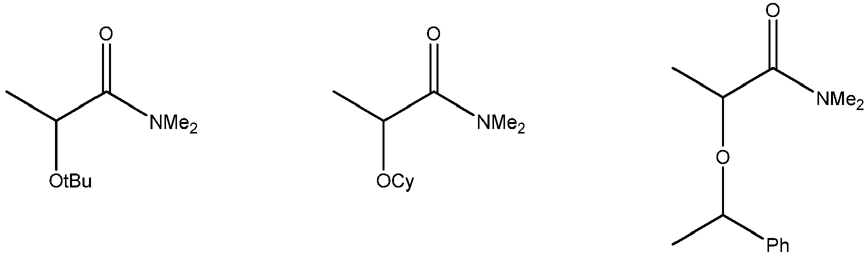

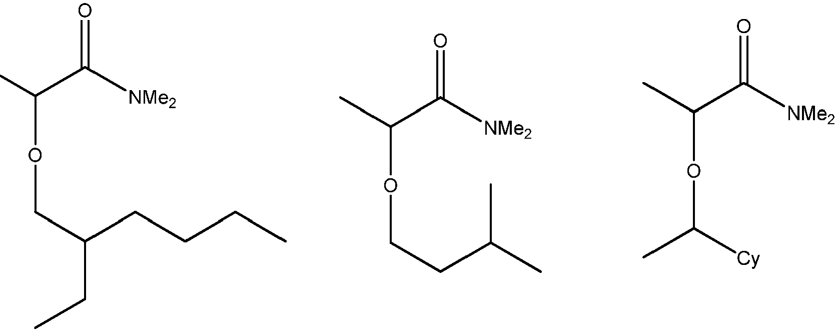

### Procédé de préparation du composé

Le composé de formule (I) peut être préparé par tout procédé approprié. Le procédé peut en outre comprendre une des étapes suivantes ou les deux:
étape a) une étape de d'amidification permettant d'obtenir le groupe - CONR¹R², et/ou
étape b) une étape de substitution ou d'addition nucléophile permettant d'obtenir le groupe -OR¹.

Les étapes a) et b) peuvent être mises en oeuvre dans l'ordre a) puis b) ou dans l'ordre b) puis a). On préfère toutefois opérer a) puis b).

Les étapes peuvent être mises en oeuvre à partir d'un produit de départ comprenant:
- un groupe R^{a}R^{b}CZ¹-, où Z¹ est un groupe nucléophile et/ou nucléofuge
- un groupe -CO-Z² où Z² est un groupe choisi parmi OR', Cl, où R' est atome d'hydrogène ou un groupe alkyle, de préférence un groupe alkyle en C₁-C₄, par exemple un groupe méthyle ou éthyle.

Des produits de départ particulièrement utiles sont les produits de formule (I') suivante:

R^{a}R^{b}CZ¹-CO-Z² (I')

On cite à titre d'exemple l'acide glycolique, le glycolate de méthyle, le glycolate d'éthyle, l'acide lactique, le lactate de méthyle, le lactate d'éthyle, le 2-chloropropanoate de méthyle. De tels produits sont disponibles dans le commerce. On mentionne que l'acide glycolique ou son ester peut être obtenu à partir de la canne à sucre, et que l'acide lactique ou son ester peut être obtenu à partir du lait. Ces produits peuvent alternativement être obtenus par voie microbiologique. Il s'agit là de matières renouvelables particulièrement appropriées.

### Etape a) amidification

L'étape a) est une étape d'amidification. Elle permet typiquement de transformer un groupe -CO-Z² en un groupe -CONR²R³. Elle est typiquement mise en oeuvre à l'aide d'une amine de formule HNR²R³.

Cette étape peut notamment être mise en oeuvre en transformant le groupe - COOH en groupe -CONR²R³ directement par réaction avec une amine de formule HNR²R³ ou en formant un groupe -COCl puis en réagissant avec une amine de formule HNR²R³, de manière à obtenir le composé de formule (I).

On peut notamment mettre en oeuvre la séquence suivante: on transforme le groupe -COOH en groupe -CONR²R³ directement par réaction avec une amine de formule HNR²R³.

On peut aussi opérer une trans-amidification. Ceci peut être mis en oeuvre de manière connue. Lors de cette réaction on met de préférence en oeuvre typiquement de 0,8 à 1,2 moles, de préférence de 0,9 à 1,1 moles, de préférence environ 1 mole, d'amine par mole d'ester. On peut notamment mettre en oeuvre des catalyseurs acides (notamment des acide de Lewis) ou basiques, par exemple à l'aide carbonate de potassium ou d'ortho titanates d'alkyles. Cette étape peut être mise en oeuvre en solution, par exemple en solution aqueuse, ou en solution dans du toluène. On peut au cours de cette étape éliminer progressivement le méthanol formé afin de favoriser la réaction. L'élimination peut s'accompagner d'une élimination du solvant, par exemple par formation d'un azéotrope. Après séparation du méthanol le solvant éliminé peut être réintroduit dans le procédé.

### Etape b) substitution ou addition nucléophile

L'étape b) est une étape de substitution ou d'addition nucléophile. Elle permet typiquement de transformer un groupe R^{a}R^{b}CZ¹- en un groupe -OR¹. Les substitutions et additions nucléophiles sont des réactions connues de l'homme du métier. Elles peuvent être mises en oeuvre en présence d'un acide ou d'une base, de manière connue. On donne quelques détails ci-dessous

### b¹) Substitution nucléophile

Dans le cadre d'une substitution nucléophile, selon un premier mode de réalisation, le groupe nucléophile est un groupe Z¹=OH, mis en présence d'un composé R¹-Y comprenant un groupe nucléofuge Y (groupe partant). A titre d'exemples de composés comprenant un groupe nucléofuge on cite par exemple les composés de formule R¹O-X-OR¹ où X est -CO- ou -SO₂-, les composés de formule R¹-X où X est d'halogène comme Br, Cl, I, les composés R¹-OH.

On peut par exemple mettre en oeuvre les schémas réactionnels suivants (où Z'² = Z² ou -NR¹R²):

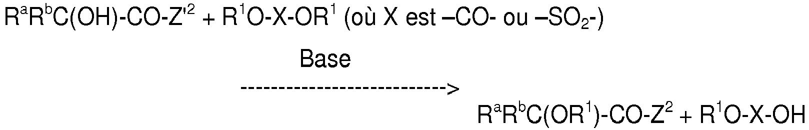

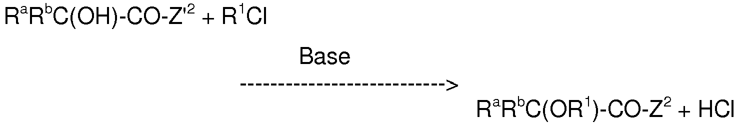

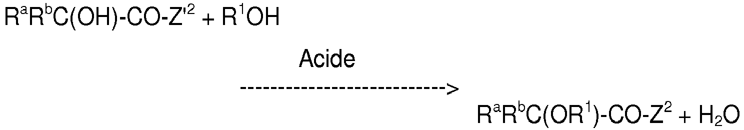

Dans le cadre d'une substitution nucléophile, selon un deuxième mode de réalisation le composé nucléophile est un alcool HOR¹ mis en présence d'un composé précurseur du composé de formule (I) et porteur d'un groupe Z¹ nucléofuge en lieu et place du futur groupe -OR¹. A titre de groupe Z¹ nucléofuge, on cite par exemple les atomes d'halogène comme Br, Cl, I et les groupes X-OR où X=CO ou SO₂ et R est un alkyle ou un groupe aromatique, par exemple un groupe mésylate ou tosylate ou carbonate. Ce mode de réalisation peut par exemple être mis en oeuvre à partir d'un composé de formule R^{a}R^{b}CCl-CO-Me, obtenu par chloration radicalaire d'un composé de formule R^{a}R^{b}CH-CO-OMe. Ce mode de réalisation peut aussi être mis en oeuvre à partir d'un composé de formule R^{a}R^{b}CCl-CONR¹R², obtenu par chloration radicalaire d'un composé de formule R^{a}R^{b}CH-CONR¹R².

### b²) Addition nucléophile

Dans le cadre d'une addition nucléophile, le groupe nucléophile est un groupe Z¹=OH, mis en présence d'un composé présentant une insaturation éthylénique (R¹ correspondant audit composé saturé).

On peut par exemple mettre en oeuvre le schéma réactionnel suivant (où Z'² = Z² ou -NR¹R²) :

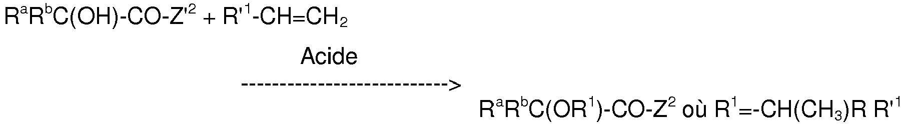

### Utilisations - Formulations

Le composé de l'invention peut notamment être utilisé comme tensioactif, solvant, co-solvant et/ou inhibiteur de cristallisation, comme agent plastifiant ou comme agent de coalescence.

Par co-solvant, on entend que d'autres solvants peuvent lui être associés. L'utilisation à titre de solvant ou de co-solvant comprend notamment des utilisations pour dissoudre un composé dans une formulation, dans un milieu réactionnel, l'utilisation pour solubiliser totalement ou partiellement un produit à éliminer (dégraissage, décapage), et/ou pour faciliter le décollage de films de matières.

Pour l'utilisation à titre de tensioactif, on préfère les composés alcoxylés et/ou propoxylés, c'est-à-dire où n est différent de 0 dans la formule (I).

Le composé de l'invention peut notamment être utilisé, pour les fonctions indiquées ci-dessus ou pour d'autres, dans une formulation phytosanitaire, dans une formulation de nettoyage, dans une formulation de décapage, dans une formulation de dégraissage, dans une formulation de lubrifiants ou textiles, dans une formulation de revêtement, par exemple dans une formulation de peinture, dans une formulation de pigments ou encre, dans une formulation plastique.

Le composé peut par exemple être utilisé à titre d'agent de coalescence dans une formulation de peinture aqueuse.

Le composé peut notamment être utilisé comme solvant de résines par exemple dans l'industrie du revêtement de câbles ou dans l'industrie électronique, notamment comme solvant du PVDF.

Le composé peut notamment être utilisé comme solvant de nettoyage et/ou de décapage dans l'industrie électronique. Il peut notamment être utilisé dans des batteries au Lithium. Il peut notamment être utilisé sur des résines photorésistantes, des polymères, des cires des graisses, des huiles.

Le composé peut notamment être utilisé pour le nettoyage d'encres, par exemple lors de la production d'encres ou lors de l'utilisation d'encre en impression.

Le composé peut notamment être utilisé pour le nettoyage de tamis ou d'autres outils mis en oeuvre dans des procédés de fabrication et/ou de recyclage de papier.

Le composé peut notamment être utilisé pour le nettoyage de bitumes ou de sables bitumineux ("tar sand" en anglais), par exemple sur les substrats revêtus, sur les outils utilisés pour appliquer ces matières, sur des vêtements souillés, sur des véhicules souillés.

Le composé peut notamment être utilisé pour le nettoyage d'engins volants comme des avions, des hélicoptères, des navettes spatiales.

Le composé peut notamment être utilisé comme agent de dégraissage sur des surfaces métalliques, par exemple des surfaces d'outils, d'objets manufacturés, de tôles, de moules, notamment en acier ou en aluminium ou en alliages de ces métaux.

Le composé peut notamment être utilisé comme solvant de nettoyage sur des surfaces dures ou des surfaces textiles.

Le composé peut notamment être utilisé comme solvant de décapage de peinture ou de résines, sur des surfaces d'outils, par exemple des moules de fonderie, sur des surfaces des sites industriels (sols, cloisons etc...). Les formulations de décapage de peintures peuvent notamment être des formulations à base aqueuse (le composé étant en mélange avec de l'eau) ou à base solvant (le composé étant alors le solvant ou un composé en mélange avec de l'eau).

Le composé peut notamment être utilisé comme agent plastifiant dans des formulations de polymères thermoplastiques.

Les formulations de nettoyage et/ou de dégraissage peuvent notamment être des formulations pour les soins ménagers, opérés dans les foyers ou dans les domaines publiques (hotels, bureaux, usines....). Il peut s'agir de formulation pour le nettoyage des surfaces dures comme les sols, les surfaces d'ameublement et d'équipement des cuisines et salles de bain, la vaisselle. Ces formulations peuvent également être utilisées dans la sphère industrielle pour dégraisser des produits manufacturés et/ou les nettoyés. De telles formulation peuvent notamment être utilisées pour nettoyer et/ou décaper des produits, des outils, des moules, des habits ou autres.

Le composé de l'invention peut notamment être utilisé dans des formulations phytosanitaires comprenant un produit actif solide. Plus de détails sont donnés ci-dessous, où le mot "solvant" peut désigner le composé de l'invention ou une composition de matière le comprenant, décrite ci-dessus.

### Utilisation détaillée dans le cadre de formulations phytosanitaires

La formulation phytosanitaire est généralement une formulation phytosanitaire concentrée comprenant un composé actif.

L'agriculture utilise de nombreuses matières actives telles que des fertilisants ou des pesticides, par exemple des insecticides, herbicides ou fongicides. On parle de produits phytosanitaires actifs (ou de matière active). Les produits phytosanitaires actifs sont généralement produits sous forme pure ou très concentrée. Ils doivent être utilisés sur les exploitations agricoles à de faibles concentrations. A cette fin, ils sont généralement formulés avec d'autres ingrédients afin de permettre une dilution en poids aisée par l'exploitant agricole. On parle de formulations phytosanitaires. La dilution opérée par l'exploitant agricole est généralement réalisée par mélange de la formulation phytosanitaire avec de l'eau.

Ainsi les formulations phytosanitaires doivent permettre une dilution en poids aisée par l'exploitant agricole, afin d'obtenir un produit dans lequel le produit phytosanitaire est correctement dispersé, par exemple sous forme de solution, d'émulsion, de suspension, ou de suspo-émulsion. Les formulations phytosanitaires permettent ainsi le transport d'un produit phytosanitaire sous forme relativement concentrée, un conditionnement aisé et/ou une manipulation aisée pour l'utilisateur final. Différents types de formulations phytosanitaires peuvent être utilisés selon les différents produits phytosanitaires. On cite par exemple les concentrés émulsionnables (Emulsifiable Concentrates «EC»), les émulsions concentrées (Emulsion in water "EW"), les microémulsions ("ME"), les poudres mouillables (Wettable Powders «WP»), les granulés dispersables dans l'eau (Water Dispersible Granules, «WDG»). Les formulations qu'il est possible d'utiliser dépendent de la forme physique du produit phytosanitaire (par exemple solide ou liquide) et de ses propriétés physico-chimiques en présence d'autres composés comme l'eau ou les solvants.

Après dilution en poids par l'exploitant agricole, par exemple par mélange avec de l'eau, le produit phytosanitaire peut se trouver sous différentes formes physiques: solution, dispersion de particules solides, dispersion de gouttelettes du produit, gouttelettes de solvant dans lequel le produit est dissous... Les formulations phytosanitaires comprennent généralement des composés permettant d'obtenir ces formes physiques. Il peut par exemple s'agir de tensioactifs, de solvants, de supports minéraux, et/ou de dispersants. Bien souvent ces composés n'ont pas un caractère actif, mais un caractère d'intermédiaire d'aide à la formulation. Les formulations phytosanitaires peuvent notamment être sous forme liquide, ou sous forme solide.

Afin de préparer des formulations phytosanitaires de produits phytosanitaires actifs solides, il est connu de solubiliser le produit dans un solvant. La formulation phytosanitaire comprend ainsi une solution du produit dans le solvant. La formulation peut être sous forme solide, par exemple sous forme de poudre mouillable (WP) où la solution imbibe un support inorganique, par exemple du kaolin et/ou de la silice. La formulation peut alternativement être sous forme liquide, par exemple sous forme de concentré émulsionnable (EC) présentant une seule phase liquide limpide comprenant le solvant et le produit en solution, pouvant former une émulsion par ajout d'eau, sans agitation ou avec une faible agitation. Elle peut aussi être ou sous forme d'une émulsion concentrée (EW), trouble, dont la phase dispersée dans l'eau comprend le solvant et le produit en solution dans le solvant. Elle peut aussi être forme d'une microémulsion (ME), limpide, dont la phase dispersée dans l'eau comprend le solvant et le produit en solution dans le solvant.

Certains actifs phytosanitaires solides sont souvent difficiles à formuler. Par exemple le tebuconazole est un fongicide particulièrement efficace, et d'utilisation répandue, pour la culture du soja notamment. Pour certains actifs phytosanitaires, il est difficile de réaliser des formulations concentrées, faciles à diluer pour l'exploitant agricole, stables, et sans inconvénients (avérés ou perçus) substantiels en matière de sécurité, de toxicité et/ou d'eco-toxicité. Pour certains actifs, il est difficile de formuler à des concentrations relativement élevées, avec une stabilité suffisante. En particulier il est nécessaire d'éviter l'apparition de cristaux en particulier à basse température et/ou lors de la dilution et/ou lors du stockage à température élevée de la composition diluée. Les cristaux peuvent avoir des effets négatifs, notamment boucher les filtres des dispositifs utiliser pour répandre la composition diluée, boucher les dispositifs de pulvérisation, diminuer l'activité globale de la formulation, créer des problèmes inutiles de filières de déchets pour éliminer les cristaux, et/ou provoquer une mauvaise répartition du produit actif sur le champ agricole.

Les formulations comprenant le solvant présentent notamment :
- une solubilisation de quantités importantes d'actifs,
- une absence de cristallisation, même des conditions exigeantes,
- une bonne activité biologique pouvant être due à une bonne solvatation, et/ou
- un profil de sécurité, toxicologie et/ou eco-toxicologie perçu comme favorable.

La formulation phytosanitaire peut en outre être une formulation phytosanitaire concentrée comprenant:
a) un produit phytosanitaire actif,
b) le solvant
c) éventuellement au moins agent émulsifiant, de préférence un tensioactif, et
d) éventuellement de l'eau.

La formulation phytosanitaire de l'invention peut notamment être sous forme d'un concentré émulsionnable (EC), d'une microémulsion concentrée de préférence dans l'eau (ME) ou d'une émulsion concentrée de préférence dans l'eau (EW).

### Produit phytosanitaire actif a)

Des produits phytosanitaires actifs, notamment des produits non solubles dans l'eau et solides sont connus de l'homme du métier. Le produit phytosanitaire actif peut notamment être un herbicide, un insecticide, un acaricide, un fongicide, ou un agent d'élimination des rongeurs ("rodenticide" en anglais) par exemple un raticide.

A titre d'exemples non limitatifs de matières actives convenables, on peut citer entre autres l'Amétryne, le Diuron, le Linuron, le Chlortoluron, l'Isoproturon, le Nicosulfuron, le Metamitron, le Diazinon, l'Aclonifen, l'Atrazine, le Chlorothalonil, le Bromoxynil, le Bromoxynil heptanoate, le Bromoxynil octanoate, le Mancozeb, la Manèbe, le Zineb, la Phenmédipham, le Propanyl, la série des phénoxyphénoxy, la série des hétéroaryloxyphénoxy, le CMPP, le MCPA, le 2,4-D, la Simazine, les produits actifs de la série des imidazolinones, la famille des organophosphorés, avec notamment l'Azinphos-éthyl, l'Azinphos-méthyl, l'Alachlore, le Chlorpyriphos, le Diclofop-méthyl, le Fénoxaprop-p-éthyl, le Méthoxychlore, la Cyperméthrine, le Fenoxycarbe, le cymoxanil, le chlorothalonyl, les insecticides neonicotinoides, la famille des fongicide triazoles tels que l'azaconazole, bromuconazole, cyproconazole, difenoconazole, diniconazole, epoxyconazole, fenbuconazole, flusilazole, myclobutanyl, tebuconazole, triadimefon, triadimenol, des strobilurines telles que la pyraclostrobine, la picoxystrobine, l'azoxystrobine, la famoxadone, le kresoxym-methyl et la trifloxystrobine, les solfonylurées telles que le bensulfuron-methyl, le chlorimuron-ethyl, le chlorsulfuron, le metsulfuron-methyl, le nicosulfuron, le sulfomethuron-methyl, le triasulfuron, le tribenuron-methyl.

On choisit parmi cette liste de préférence les produits non-hydrosolubles.

On peut notamment mettre en oeuvre les produits phytosanitaires actifs suivants :

| | |
|---|---|
| Alachlor | 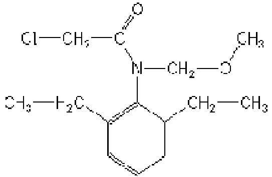 |
| Chlorpyrifos | 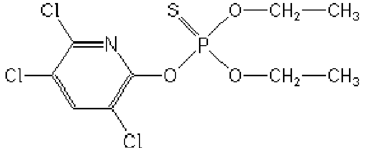 |
| alpha-cyperméthrine | 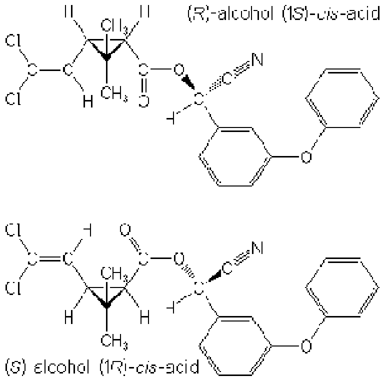 |
| | En mélange racémique et/ou en stéréoisomères isolés. |
| Phenmedipham | 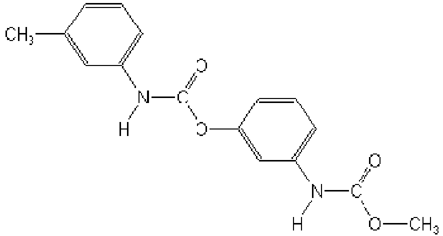 |
| Propanil | 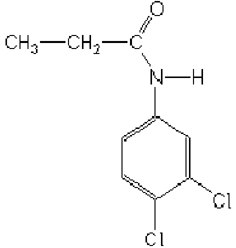 |
| Pendimethalin | 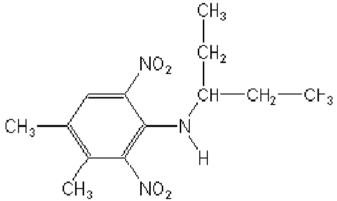 |
| triadimenol | 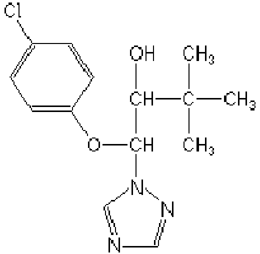 |
| Trifluralin | 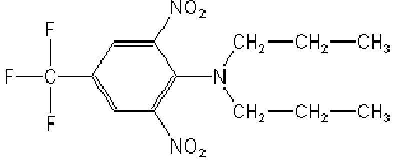 |
| Oxyfluorfen | 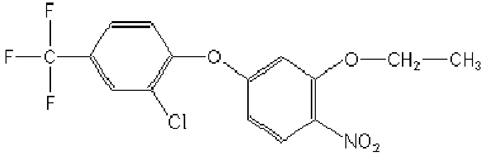 |
| Dimethoate | 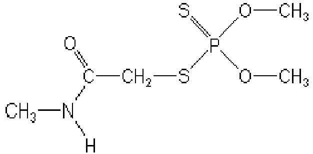 |
| Imidacloprid | 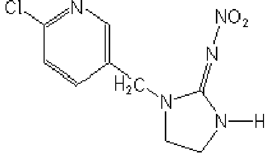 |
| Proxopur | 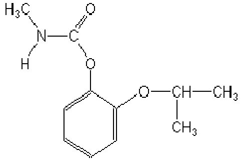 |
| Benomyl | 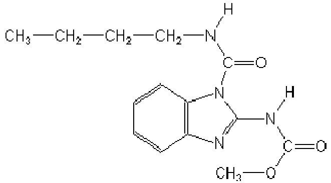 |
| Deltamethrine | 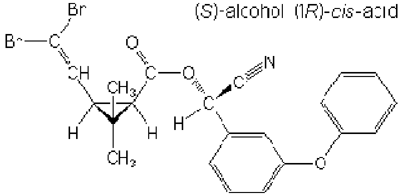 |
| Fenvalerate | 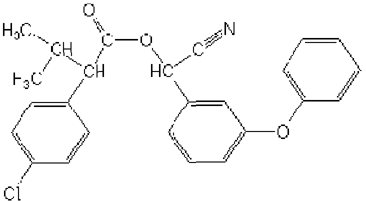 |
| Abamectin | 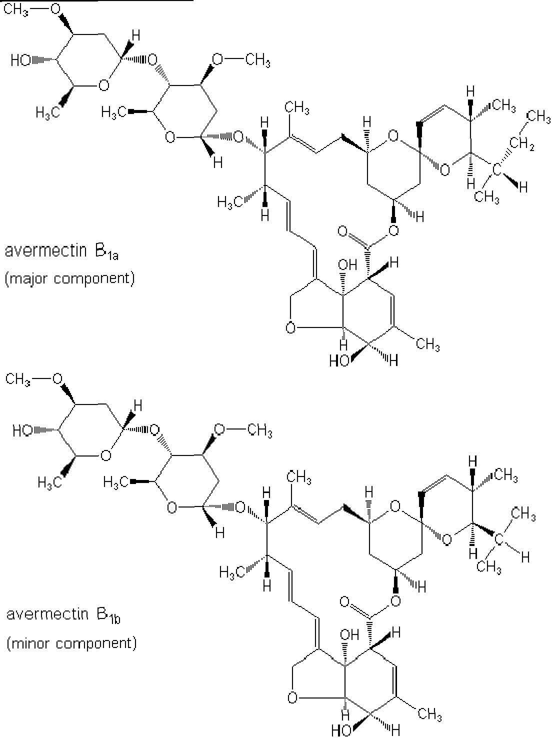 |
| Amicarbazone | 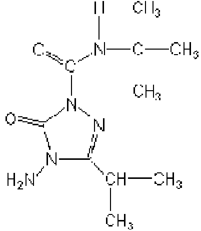 |
| Bifenthrin | 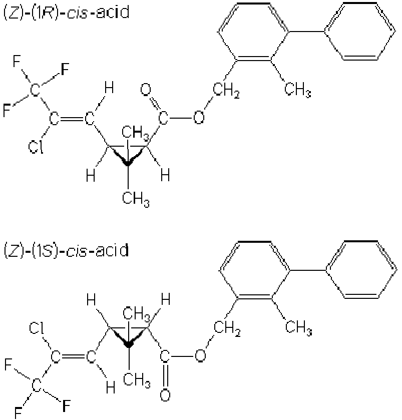 |
| Carbosulfan | 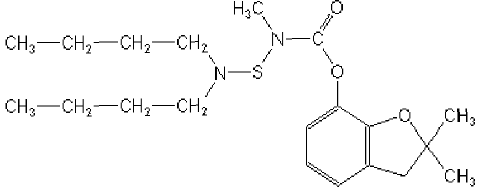 |
| Cyfluthrin | 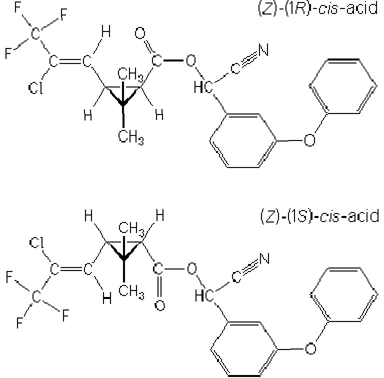 |
| Difenconazole | 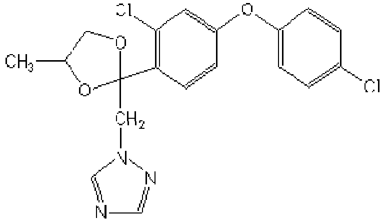 |
| Ethofenprox | 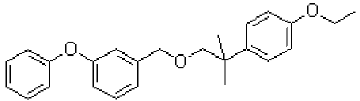 |
| Fenoxaprop-ethyl | 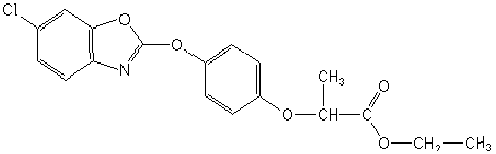 |
| Fipronil | 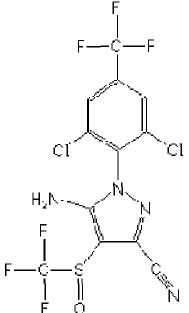 |
| Fenvalerate | 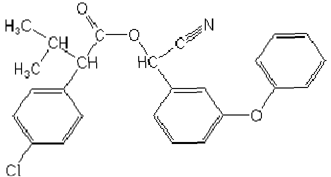 |
| Fluazifop-p-butyl | 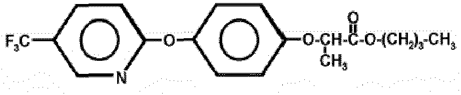 |
| Flufenouron | 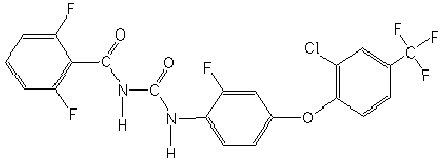 |
| Hexazinone | 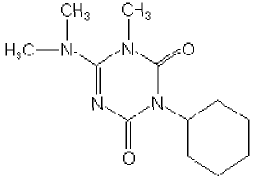 |
| Lambda-cyalothrin | 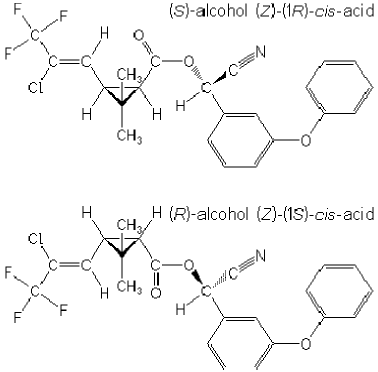 |
| Methomyl | 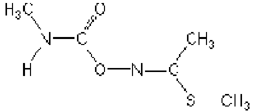 |
| Permethrin | 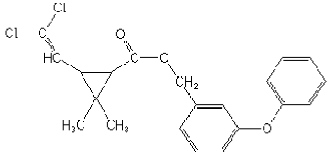 |
| Prochloraz | 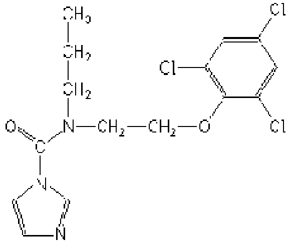 |
| Propiconazole | 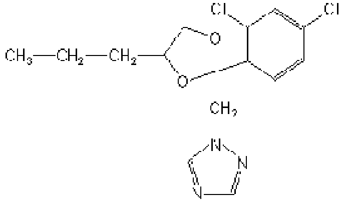 |
| Tebuconazole | 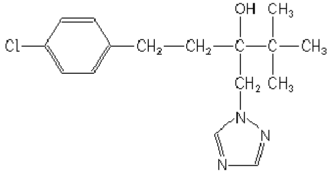 |

Ces produits et dénominations sont connus de l'homme du métier. On peut associer plusieurs produits phytosanitaires actifs.

### Agent émulsifiant c)

La formulation phytosanitaire peut comprendre un agent émulsifiant, typiquement et de préférence un tensioactif. Les agents émulsifiants sont des agents destinés à faciliter la mise en émulsion ou la dispersion après mise en présence de la formulation avec de l'eau, et/ou à stabiliser (dans le temps et/ou en température) l'émulsion ou la dispersion, par exemple en évitant une sédimentation.

Les tensioactifs sont des composés connus, qui présentent une masse molaire généralement relativement faible, par exemple inférieure à 1000 g/mol. Le tensioactif peut être un tensioactif anionique sous forme salifiée ou acide, non ionique de préférence polyalcoxylé, cationique, amphotère (terme incluant aussi les tensioactifs zwitterioniques). Il peut s'agir d'un mélange ou d'une association de ces tensioactifs.

A titre d'exemples de tensioactifs anioniques, on peut citer, sans intention de s'y limiter :
- les acides alkylsulfoniques, les acides arylsulfoniques, éventuellement substitués par un ou plusieurs groupements hydrocarbonés, et dont la fonction acide est partiellement ou totalement salifiée, comme les acides alkylsulfoniques en C₈-C₅₀, plus particulièrement en C₈-C₃₀, de préférence en C₁₀-C₂₂, les acides benzènesulfoniques, les acides naphtalènesulfoniques, substitués par un à trois groupements alkyles en C₁-C₃₀, de préférence en C₄-C₁₆, et/ou alcényles en C₂-C₃₀, de préférence en C₄-C₁₆.
- les mono- ou diesters d'acides alkylsulfosucciniques, dont la partie alkyle, linéaire ou ramifiée, éventuellement substituée par un ou plusieurs groupements hydroxylés et/ou alcoxylés, linéaires ou ramifiés en C₂-C₄ (de préférence éthoxylés, propoxylés, éthopropoxylés).
- les esters phosphates choisis plus particulièrement parmi ceux comprenant au moins un groupement hydrocarboné saturé, insaturé ou aromatique, linéaire ou ramifié, comprenant 8 à 40 atomes de carbone, de préférence 10 à 30, éventuellement substitués par au moins un groupement alcoxylé (éthoxylé, propoxylé, éthopropoxylé). En outre, ils comprennent au moins un groupe ester phosphate, mono- ou diestérifié de telle sorte que l'on puisse avoir un ou deux groupes acides libres ou partiellement ou totalement salifiés. Les esters phosphates préférés sont du type des mono- et diesters de l'acide phosphorique et de mono-, di- ou tristyrylphénol alcoxylé (éthoxylé et/ou propoxylé), ou de mono-, di- ou trialkylphénol alcoxylé (éthoxylé et/ou propoxylé), éventuellement substitué par un à quatre groupements alkyles ; de l'acide phosphorique et d'un alcool en C₈-C₃₀, de préférence en C₁₀-C₂₂ alcoxylé (éthoxylé ou éthopropoxylé); de l'acide phosphorique et d'un alcool en C₈-C₂₂, de préférence en C₁₀-C₂₂, non alcoxylé.
- les esters sulfates obtenus à partir d'alcools saturés, ou aromatiques, éventuellement substitués par un ou plusieurs groupements alcoxylés (éthoxylés, propoxylés, éthopropoxylés), et pour lesquels les fonctions sulfates se présentent sous la forme acide libre, ou partiellement ou totalement neutralisées. A titre d'exemple, on peut citer les esters sulfates obtenus plus particulièrement à partir d'alcools en C₈-C₂₀, saturés ou insaturés, pouvant comprendre 1 à 8 motifs alcoxylés (éthoxylés, propoxylés, éthopropoxylés) ; les esters sulfates obtenus à partir de phénol polyalcoxylé, substitués par 1 à 3 groupements hydroxycarbonés en C₂-C₃₀, saturés ou insaturés, et dans lesquels le nombre de motifs alcoxylés est compris entre 2 et 40; les esters sulfates obtenus à partir de mono-, di- ou tristyrylphénol polyalcoxylés dans lesquels le nombre de motifs alcoxylés varie de 2 à 40.

Les tensioactifs anioniques peuvent être sous forme acide (ils sont potentiellement anioniques), ou sous une forme partiellement ou totalement salifiée, avec un contre-ion. Le contre-ion peut être un métal alcalin, tel que le sodium ou le potassium, un alcalino-terreux, tel que le calcium, ou encore un ion ammonium de formule N(R)₄⁺ dans laquelle R, identiques ou différents, représentent un atome d'hydrogène ou un radical alkyle en C₁-C₄ éventuellement substitué par un atome d'oxygène.

A titres d'exemples de tensioactifs non ioniques, on peut citer, sans intention de s'y limiter:
- les phénols polyalcoxylés (éthoxylés, propoxylés, éthopropoxylés) substitués par au moins un radical alkyle en C₄-C₂₀, de préférence en C₄-C₁₂, ou substitués par au moins un radical alkylaryle dont la partie alkyle est en C₁-C₆. Plus particulièrement, le nombre total de motifs alcoxylés est compris entre 2 et 100. A titre d'exemple, on peut citer les mono-, di- ou tri (phényléthyl) phénols polyalcoxylés, ou les nonylphénols polyalcoxylés. Parmi les di- ou tristyrylphenols éthoxylés et/ou propoxylés, sulfatés et/ou phosphatés, on peut citer, le di-(phényl-1 éthyl)phénol éthoxylé, contenant 10 motifs oxyéthylénés, le di-(phényl-1 éthyl)phénol éthoxylé, contenant 7 motifs oxyéthylénés, le di-(phényl-1 éthyl)phénol éthoxylé sulfaté, contenant 7 motifs oxyéthylénés, le tri-(phényl-1 éthyl)phénol éthoxylé, contenant 8 motifs oxyéthylénés, le tri-(phényl-1 éthyl)phénol éthoxylé, contenant 16 motifs oxyéthylénés, le tri-(phényl-1 éthyl)phénol éthoxylé sulfaté, contenant 16 motifs oxyéthylénés, le tri-(phényl-1 éthyl)phénol éthoxylé, contenant 20 motifs oxyéthylénés, le tri-(phényl-1 éthyl)phénol éthoxylé phosphaté, contenant 16 motifs oxyéthylénés.
- les alcools ou les acides gras en C₆-C₂₂, polyalcoxylés (éthoxylés, propoxylés, éthopropoxylés). Le nombre des motifs alcoxylés est compris entre 1 et 60. Le terme acide gras éthoxylé inclut aussi bien les produits obtenus par éthoxylation d'un acide gras par l'oxyde d'éthylène que ceux obtenus par estérification d'un acide gras par un polyéthylèneglycol.
- les triglycérides polyalcoxylés (éthoxylés, propoxylés, éthopropoxylés) d'origine végétale ou animale. Ainsi conviennent les triglycérides issus du saindoux, du suif, de l'huile d'arachide, de l'huile de beurre, de l'huile de graine de coton, de l'huile de lin, de l'huile d'olive, de l'huile de palme, de l'huile de pépins de raisin, de l'huile de poisson, de l'huile de soja, de l'huile de ricin, de l'huile de colza, de l'huile de coprah, de l'huile de noix de coco, et comprenant un nombre total de motifs alcoxylés compris entre 1 et 60. Le terme triglycéride éthoxylé vise aussi bien les produits obtenus par éthoxylation d'un triglycéride par l'oxyde d'éthylène que ceux obtenus par trans-estérification d'un triglycéride par un polyéthylèneglycol.
- les esters de sorbitan éventuellement polyalcoxylés (éthoxylés, propoxylés, éthopropoxylés), plus particulièrement les esters de sorbitol cyclisé d'acides gras de C₁₀ à C₂₀ comme l'acide laurique, l'acide stéarique ou l'acide oléique, et comprenant un nombre total de motifs alcoxylés compris entre 2 et 50.

Des émulsifiants utiles sont notamment les produits suivants, tous commercialisés par Rhodia :
- Soprophor® TSP/724: tensioactif à base de tristyrylphonol éthopropoxylé
- Soprophor® 796/O: tensioactif à base de tristyrylphonol éthopropoxylé
- Soprophor® CY 8: tensioactif à base de tristyrylphonol éthoxylé
- Soprophor® BSU: tensioactif à base de tristyrylphonol éthoxylé
- Alkamuls® RC: tensioactif à base d'huile de ricin éthoxylée
- Alkamuls® OR/36: tensioactif à base d'huile de ricin éthoxylée
- Alkamuls® T/20: tensioactif à base d'un ester de sorbitan

La formulation comprend avantageusement au moins 4%, de préférence au moins 5%, de préférence au moins 8%, en poids de matière sèche, d'au moins un tensioactif c).

On mentionne que le solvant peut être associé à un tensioactif aromatique et/ou non aromatique.

### Autres détails quant à la formulation phytosanitaire

La formulation phytosanitaire, concentrée, ne comprend de préférence pas des quantités importantes d'eau. Typiquement la teneur en eau est inférieure à 50% en poids, avantageusement inférieure à 25% en poids. Elle sera généralement inférieure à 10% en poids.

La formulation est de préférence un formulation liquide, par exemple sous forme d'un concentré emulsifiable (EC), d'une émulsion concentrée (EW) ou d'une micorémulsion (ME). Dans ce cas elle comprend de préférence moins de 500 g/L d'eau, plus préférablement moins de 250 g/L. Elle sera généralement inférieure à 100 g/L.

Les formulations peuvent avantageusement comprendre:
a) de 4 à 60%, de préférence de 10 à 50%, du produit phytosanitaire, en poids de matière active,
b) de 10 à 92%, de préférence de 20 à 80%, du solvant, en poids,
c) de 4 à 60%, de préférence de 5 à 50%, de préférence de 8 à 25%, en poids de matière sèche, d'un émulsifiant, de préférence d'un tensioactif,
d) de 0 à 10% en poids d'eau.

Il n'est pas exclu de réaliser des formulations solides, par exemple des formulations dans lesquelles un liquide comprenant le produit phytosanitaire solubilisé dans le solvant, est supporté par un minéral et/ou dispersé dans une matrice solide.

La formulation peut bien entendu comprendre d'autres ingrédients (ou "autres additifs") que le produit phytosanitaire actif, le(s) solvant(s), le(s) agent(s) émulsifiant(s) optionnel(s) et l'eau optionnelle. Elle peut notamment comprendre des agents de modification de la viscosité, des agents antimousse, notamment des antimousse siliconnés, des agents anti-rebond, des agents anti-lessivage, des charges inertes, notamment des charges minérales, des agents anti-gel...

Notamment les formulations peuvent comprendre des additifs, dits autres additifs, ne rentrant pas dans la définition des produits a), b), ou c), comme:
- d'autres solvants, généralement en faible quantité, c'est-à-dire en quantité inférieure au solvant du système solvant étant présent dans la plus faible quantité. Un solvant autre n'est pas entendu comme faisant partie du système solvant. A titre phosphonates ou des oxydes de phosphines comme le TEBP, le TBP, le TEPO, le DBBP. On cite également les alkyldiméthyleamides où l'alkyl est en C₆-C₁₈, notamment ceux commercialisés sous la marque Genagen. On cite également les lactates d'esters, notamment ceux commercialisés sous la marque Purasolv. On cite également les esters méthyliques d'acides gras, notamment ceux commercialisés sous la marque Phytorobe. On cite également les diesters de diacides ("DiBasic Esters" en anglais), notamment ceux commercialisé par Rhodia sous les marques Rhodiasolv RPDE, et Rhodiasolv DIB. On cite également les coupes d'hydrocarbures, les amides cycliques comme la NMP, les lactones. On cite également les bis(dialkylamides) décrites dans le document WO 2008/074837.

- des inhibiteurs de cristallisation. Il peut s'agir des solvants mentionnés ci-dessus. Il peut également s'agir d'acides gras ou d'alcools gras non polyalkoxylés. On cite par exemple le produit Alkamuls® OL700 commercialisé par Rhodia.

Des procédés classiques de préparation de formulations phytosanitaires ou de mélanges de solvants peuvent être mis en oeuvre. On peut opérer par simple mélange des constituants.

La formulation phytosanitaire concentrée est destinée à être répandue sur un champ cultivé ou à cultiver, par exemple un champ de soja, le plus souvent après dilution dans de l'eau, pour obtenir une composition diluée. La dilution est généralement opérée par l'exploitant agricole, directement dans un réservoir ("tank-mix"), par exemple dans le réservoir d'un dispositif destiné à répandre la composition. Il n'est pas exclu que l'exploitant ajoute d'autres produits phytosanitaires, par exemple des fongicides, herbicides, pesticides, insecticides, des fertilisants. Ainsi, la formulation peut être utilisée pour préparer une composition diluée dans l'eau du produit phytosanitaire actif, par mélange d'au moins une part en poids de formulation concentrée avec au moins 10 parts d'eau, de préférence moins de 1000 parts. Les taux de dilution et les quantités à appliquer sur le champ dépendent généralement du produit phytosanitaire et de la dose souhaitable pour traiter le champ; cela peut être déterminé par l'exploitant agricole.

### EXEMPLES

### Exemple 1.1 - Préparation de CH₃- CH(O-Me)-CONMe₂

La voie de synthèse est la suivante :

### Première Etape

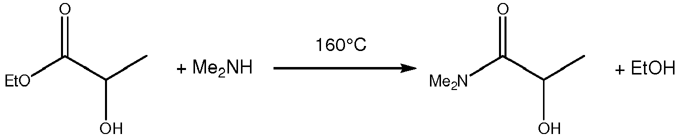

### Deuxième Etape

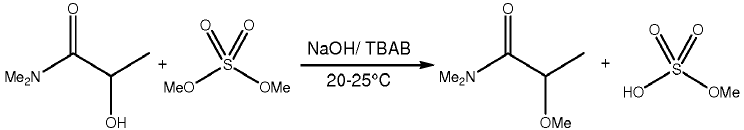

### Première étape :

Dans un autoclave de 2 L, sont mélangés à +25°C le lactate d'éthyle (802g, 6.79 mol, 1 équivalent) et la N,N-diméthylamine (380g, 8046 mol, 1.2 équivalent). Le mélange est chauffé à +160°C sous pression pendant 16 heures. Le brut réactionnel est purifié par distillation sous pression réduite (P <1 mbar, T vapeur = 55-56°C). Le diméthyl-lactamide (538 g) est ainsi obtenu avec une pureté > 99%. Ceci correspond à un rendement de l'ordre de 69-70%.

### Deuxième étape :

Dans un ballon de 3 L, sont mélangés à +25°C la soude 50% dans l'eau (240 g, 3 mol, 1.5 équivalents), le dichlorométhane (1L), le diméthylsulfate (378 g, 3 mol, 1.5 équivalents) et le bromure de tétrabutylammonium (0.5 g, 1.6 mmol, 0.0004 équivalent). A ce mélange est additionné en 1 heure la diméthyl-lactamide (234 g, 2 mol, 1 équivalent) à +20°C. Le mélange est maintenu sous agitation forte à cette température jusqu'à consommation complète du diméthyl-lactamide (16 heures). Le milieu est dilué avec de l'eau (0.5L). La phase organique est alors séparée de l'eau par décantation et la phase aqueuse résiduelle est extraite 3 fois par 0.5 L de dichlorométhane. Les phases organiques sont rassemblées et le solvant évaporé sous vide réduit. Le brut réactionnel est purifié par distillation sous pression réduite (P <1 mbar, T vapeur = 48-50°C). Le produit désiré (538 g) est ainsi obtenu avec une pureté > 99%. Ceci correspond à un rendement de l'ordre de 89 %.

### Exemple 1.2 - Préparation de CH₃-CH(O-Cy)-CONMe₂

La voie de synthèse est la suivante :

### Première Etape

Identique à la première étape de l'exemple 1.

### Deuxième Etape

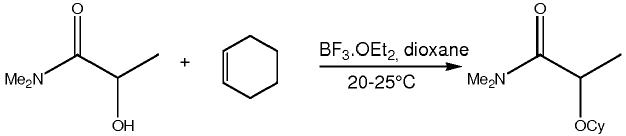

Dans un ballon de 2 L, sont mélangés à +25°C l'éthérate de trifluorure de bore (396 g, 2.79 mol, 3 équivalents) et le dioxane (0.5L). A ce mélange est additionné en 1 heure la diméthyl-lactamide (109 g, 0.93 mol, 1 équivalent) à +4°C. Le mélange est progressivement réchauffé à +40°C puis le cyclohexène (229 g, 2.79, 3 équivalents) est additionné en 10 heures sous agitation forte à cette température. Le milieu ré »actionnel est maintenu à cette température jusqu'à consommation complète du diméthyl-lactamide (24 heures). Le milieu, refroidi à +20°C, est traité par ajout de soude 2N jusqu'à atteindre une consigne de pH comprise entre 6 et 7 (1.2L). La phase organique est alors séparée par décantation et la phase aqueuse résiduelle est extraite 3 fois par 0.75 L de dichlorométhane. Les phases organiques sont rassemblées et le solvant évaporé sous vide réduit. Le brut réactionnel est purifié par distillation sous pression réduite (P <1 mbar, T vapeur = 70-74°C). Le produit désiré (126 g) est ainsi obtenu avec une pureté > 98%. Ceci correspond à un rendement de l'ordre de 68 %.

### Exemple 1.3 - Préparation de CH₃-CH(O-Ethylhexyl)-CONMe₂

La voie de synthèse est la suivante :

### Première Etape

Identique à la première étape de l'exemple 1.

### Deuxième Etape

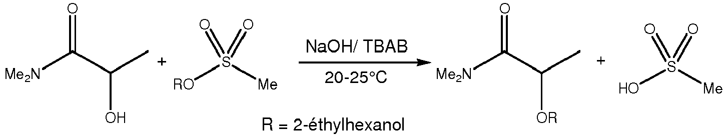

Dans un ballon de 3 L, sont mélangés à +25°C la soude 50% dans l'eau (248 g, 3.1 mol, 1.5 équivalents), le dichlorométhane (1L), le mésylate du 2-éthylhexanol (préalablement préparé par mésylation du 2-éthylhexanol) (642 g, 3.1 mol, 1.5 équivalents) et le bromure de tétrabutylammonium (0.5 g, 1.6 mmol, 0.0004 équivalent). A ce mélange est additionné en 1 heure la diméthyl-lactamide (241 g, 2.05 mol, 1 équivalent) à +20°C. Le mélange est maintenu sous agitation forte à cette température jusqu'à consommation complète du diméthyl-lactamide (16 heures). Le milieu est dilué avec de l'eau (0.5L). La phase organique est alors séparée de l'eau par décantation et la phase aqueuse résiduelle est extraite 3 fois par 0.5 L de dichlorométhane. Les phases organiques sont rassemblées et le solvant évaporé sous vide réduit. Le brut réactionnel est purifié par distillation sous pression réduite (P <1 mbar). Le produit désiré (400 g) est ainsi obtenu avec une pureté > 99%. Ceci correspond à un rendement de l'ordre de 85 %.

| Molécule | Solubilité dans l'eau (%p/p) |
|---|---|
| Exemple 1.1 | Soluble en toutes proportions |
| Exemple 1.2 | 0,4 |
| Exemple 1.3 | <0,06 |

### Exemples 2 à 4 - Utilisations comme solvants des composés des exemples 1.1 à 1.3 - Formulations phytosanitaires

Par mélange des ingrédients, on prépare des formulations de divers actifs phytosanitaires, de type concentré émulsionnable (EC).

Les formulations comprennent :
- l'actif, en quantité en poids (de matière active) indiquée dans le tableau ci-dessous,
- 10% en poids de tensioactif Alkamuls® RC, commercialisé par Rhodia
- et, comme solvant, le reste de composé des exemples.

Les exemples 2 sont des exemples comparatifs où est utilisé comme solvant le produit Rhodiasolv® ADMA10, ou Rhodiasolv® ADMA810, Rhodia (zone Asie Pacifique): Solvants alkyldiméthylamide.

On effectue les tests suivants :
- Observation visuelle à 25°C - On note l'aspect de la formulation et on repère éventuellement la présence de cristaux
- Observation visuelle à 0°C - On place la formulation pendant 7 jours à 0°C et on note l'aspect de la formulation et on repère éventuellement la présence de cristaux (test CIPAC MT39)
- Observation visuelle à 0°C avec nucléation: On introduit un cristal de la matière active dans la formulation ayant passé 7 jours à 0°C pour nucléation, et on place à nouveau la formulation pendant 7 jours à 0°C. On note l'aspect de la formulation et on repère éventuellement la présence de cristaux.

| Exemple | Solvant | Actif | Apparence à 25°C | Apparence à 0°C | Apparence à 0°C avec nucléation |
|---|---|---|---|---|---|
| 2.1C | Rhodiasolv® ADMA 10 | Alachlor - 48% | Limpide | Cristaux | Cristaux |
| 2.9C | Rhodiasolv® ADMA 10 | Trifluralin - 40% | Limpide | Limpide | Cristaux |
| 2.10C | Rhodiasolv® ADMA 10 | Difenconazole - 25% | Limpide | Limpide | Cristaux |
| 2.12C | Rhodiasolv® ADMA 10 | Dimethoate - 40% | Trouble | Trouble | Cristaux |
| 2.13C | Rhodiasolv® ADMA 10 | Oxyfluorfen - 22% | Limpide | Limpide | Cristaux |
| 2.14C | Rhodiasolv® ADMA 10 | Propoxur - 20% | Limpide | Limpide | Cristaux |
| 2.15C | Rhodiasolv® ADMA 810 | Azoxystrobin - 25% | Non soluble | Non soluble | Non soluble |
| 3.1 | Exemple 1.1 | Alachlor - 48% | Limpide | Limpide | Cristaux |
| 3.2 | Exemple 1.1 | Chlorpyrifos - 40% | Limpide | Limpide | Limpide |
| 3.3 | Exemple 1.1 | Alpha-Cypermethrine - 10% | Limpide | Limpide | Limpide |
| 3.4 | Exemple 1.1 | Phenmedipham 10% | Limpide | Limpide | Limpide |
| 3.5 | Exemple 1.1 | Propanil - 36% | Limpide | Limpide | Limpide |
| 3.7 | Exemple 1.1 | Tebuconazol - 25% | Limpide | Limpide | Limpide |
| 3.9 | Exemple 1.1 | Trifluralin - 40% | Limpide | Limpide | Limpide |
| 3.10 | Exemple 1.1 | Difenconazole - 25% | Limpide | Limpide | Limpide |
| 3.12 | Exemple 1.1 | Dimethoate - 40% | Limpide | Limpide | Limpide |
| 3.13 | Exemple 1.1 | Oxyfluorfen - 22% | Limpide | Limpide | Limpide |
| 3.14 | Exemple 1.1 | Propoxur - 20% | Limpide | Limpide | Limpide |
| 3.15 | Exemple 1.1 | Azoxystrobin - 25% | Limpide | Limpide | Limpide |
| 4.1 | Exemple 1.2 | Alachlor - 48% | Limpide | Limpide | Limpide |
| 4.7 | Exemple 1.2 | Tebuconazol - 25% | Limpide | Limpide | Limpide |
| 4.9 | Exemple 1.2 | Trifluralin - 40% | Limpide | Limpide | Limpide |
| 4.10 | Exemple 1.2 | Difenconazole - 25% | Limpide | Limpide | Limpide |
| 4.13 | Exemple 1.2 | Oxyfluorfen - 22% | Limpide | Limpide | Limpide |

### Exemple 5 - Préparation de composés 'linéaires'

La voie de synthèse est la suivante :

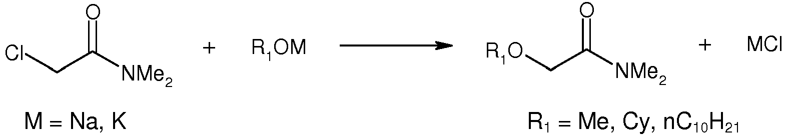

### Exemple 5.1. - Préparation de CH₃-O-CH₂-CONMe₂

### (produit 1 - R¹ = Me)

Dans un réacteur de 1 litre, initialement chargé avec du méthylate de sodium en solution dans le méthanol (25% p/p) (682 g, 3,2 mole), sous inertage d'azote, sont coulés en 2 heures le chlorodiméthylacétamide (384 g, 3,2 mole) de telle sorte que la température du milieu réactionnel n'excède pas +40°C. A la fin de la coulée, la température du milieu réactionnel est maintenue à +40°C pendant 2 heures. Après retour de la température du milieu réactionnel à +25°C, les sels formés sont retirés du mélange par filtration et les solvants sont distillés sous vide partiel. Le produit désiré 760 g est alors obtenu.

### Exemple 5.2. - Préparation de Cy-O-CH₂-CONMe₂

### (produit 2 - R¹ = Cy)

Dans un réacteur de 250 mL sont chargés le cyclohexanol (12,2 g, 0.11 mol) et le toluène (42,2 g). La température de ce mélange est portée à 30°C. La soude (5,6 g, 0,14 mol) est ensuite introduite par portions d'environ 2 g. Le mélange réactionnel est agité mécaniquement. Après dix minutes de maintien de l'agitation à 30°C, le chlorodiméthylacétamide (11,6 g, 0,09 mol) est additionné goutte à goutte directement dans la masse en 0,5 heures. Le milieu réactionnel est maintenu sous agitation à 30°C pendant 5 heures. De l'eau distillée est introduite jusqu'à ce que la totalité des sels soit dissoute (environ 30 mL). La phase organique est récupérée et les solvants sont distillés sous pression partielle et le produit désiré est obtenu 12 g avec un rendement de 72%.

### Exemple 5.3. - Préparation de nC₁₀H₂₁-O-CH₂-CONMe₂

### (produit 3 - R¹ = nC₁₀H₂₁)

Dans un réacteur de 250 mL, sous atmosphère d'azote est introduit l'hydrure de sodium (60% en suspension dans l'huile minérale) (5 g, 125 mmole). Ce produit est lavé par du pentane (3 x 15 mL) et puis mis en suspension dans du THF (100 g). La suspension est refroidie à 0°C. Le n-décanol (17,4 g, 110 mmole) est introduit avec une seringue. Le chlorodiméthylacétamide (12,1 g, 100 mmole) est alors additionné lentement sur le mélange. La température du mélange réactionnel est ramenée à l'ambiante et le mélange réactionnel est maintenu sous agitation pendant 20 heures. Le mélange réactionnel est neutralisé avec de l'eau froide (20 mL) puis cette phase aqueuse est extraite par du dichlorométhane (200 mL). La phase organique est lavée avec une solution saturée de NH₄Cl (50 mL), une solution saturée de NaHCO₃ (50 mL) puis de l'eau (50 mL). La phase organique est séchée sur sulfate de sodium et concentrée sous pression réduite pour donner 20,6 g du produit attendu avec une pureté > 98% soit un rendement de 82%.

| Molécule | Solubilité dans l'eau (%p/p) |
|---|---|
| Produit 1 | comprise entre 1 et 5 |
| Produit 2 | <0,1 |
| Produit 3 | <0,1 |

### Exemple 6 - Pouvoir solvant des principes actifs agro

### Produit 1

| Formulations | @ 25°C | @ 0°C | @ 0°C avec nucléation |
|---|---|---|---|
| Alachlor 48% EC | Limpide | Limpide | Limpide |
| Chlorpyrifos 40% EC | Limpide | Limpide | Limpide |
| Alpha-cypermethrine 10% EC | Limpide | Limpide | Limpide |
| Phenmedipham 16% EC | Limpide | Limpide | Limpide |
| Propanil 36% EC | Limpide | Limpide | Limpide |
| Pendimethalin 33% EC | Limpide | Cristal | **** |
| Tebuconazole 25% EC | Limpide | Limpide | Limpide |
| Triadimenol 23% EC | Voilé | Voilé | Voilé |
| Trifluralin 40% EC | Limpide | Limpide | Limpide |
| Difenconazole 25% EC | Limpide | Limpide | Limpide |
| Imidacloprid 20% EC | Limpide | Cristal | **** |
| Oxvfluorfen 22% EC | Limpide | Limpide | Limpide |
| Propuxur 20% EC | Limpide | Limpide | Limpide |

### Produit 2

| Formulations | @ 25°C | @ 0°C | @ 0°C avec nucléation |
|---|---|---|---|
| Alachlor 48% EC | Limpide | Limpide | Cristal |
| Chlorpyrifos 40% EC | Limpide | Limpide | Limpide |
| Alpha-Cypermethrine 10% EC | Limpide | Limpide | Limpide |
| Phenmedipham 16% EC | Limpide | Limpide | Limpide |
| Propanil 36% EC | Limpide | Limpide | Limpide |
| Pendimethalin 33% EC | Limpide | Cristal | = |
| Tebuconazole 25% EC | Limpide | Limpide | Limpide |
| Triadimenol 23% EC | Limpide | Voilé | Limpide |
| Trifluralin 40% EC | Limpide | Limpide | Limpide |
| Difenconazole 25% EC | Limpide | Limpide | Limpide |
| Oxyfluorfen 22% EC | Limpide | Limpide | Limpide |
| Propuxur 20% EC | Limpide | Limpide | Limpide |

### Produit 3

| Formulations | @ 25°C | @ 0°C | @ 0°C avec nucléation |
|---|---|---|---|
| Alachlor 48% EC | Limpide | Cristal | = |
| Chlorpyrifos 40% EC | Limpide | Limpide | Limpide |
| Phenmedipham 16% EC | Limpide | Limpide | Limpide |
| Propanil 36% EC | Limpide | Cristal | = |
| Tebuconazole 25% EC | Limpide | Limpide | Limpide |
| Triadimenol 23% EC | Voilé | Cristal | = |
| Trifluralin 40% EC | Limpide | Limpide | Limpide |
| Difenconazole 25% EC | Limpide | Limpide | Limpide |
| Oxyfluorfen 22% EC | Limpide | Cristal | = |

### Exemple 7 - Préparation de composés 'ramifiés'

### Exemple 7.1. - R^{a}=n-butyle et R^{b}=H et R¹ = Me (produit 4)

### Etape 1 :

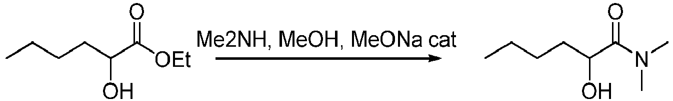

Dans un tricol, sont mélangés à 20°C et sous atmosphère inerte le 2-hydroxycaproate d'éthyle (24,24 g; 150 mmole) et le méthylate de sodium en solution dans le méthanol (25% p/p) (1,6g ; 7 mmole). A ce mélange est additionnée la diméthylamine en solution dans le méthanol (50% p/p) (26,75 g ; 297 mmole) en 30 minutes. La température de ce mélange est portée et maintenue pendant 3 heures à 40°C. Après retour à 20°C de la température de ce mélange réactionnel, de l'acide orthophosphorique est additionné jusqu'à obtenir un pH de 5. Les sels formés sont retirés par filtration et les solvants sont retirés sous pression réduite. Le 2-hydroxy-N,N-diméthylhexanamide est alors obtenu une pureté de 98% soit un rendement de 81%.

### Etape 2 :

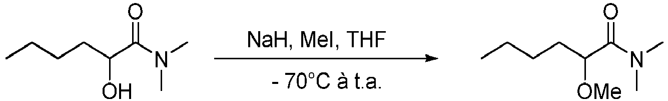

Dans un tricol, muni d'une ampoule à addition isobare, d'une entrée d'azote et de deux septa est introduit l'hydrure de sodium (60% en suspension dans l'huile minérale) (1,32 g, 33 mmole). Ce produit est lavé par du pentane (3 x 15 mL) et puis mis en suspension dans le THF (148 g). La suspension est refroidie à -70°C. Le 2-hydroxy-N,N-diméthylhexanamide (4,45g, 30 mmole) est introduit avec une seringue. L'iodométhane (5,89g, 41 mmole) est alors additionné lentement sur le mélange. La température du mélange réactionnel est ramenée à l'ambiante et le mélange réactionnel est maintenu sous agitation pendant 20 heures. Le mélange réactionnel est neutralisé avec de l'eau froide (20 mL) puis cette phase aqueuse est extraite par de l'éther diéthylique (200 mL). La phase organique est lavée avec une solution saturée de NH₄Cl (50 mL), une solution saturée de NaHCO₃ (50 mL) puis de l'eau (50 mL). La phase organique est séchée sur sulfate de sodium et concentrée sous pression réduite pour donner 3,75 g du produit attendu avec une pureté > 98% soit un rendement de 77%.

### Exemple 7.2. - R^{a}= n-butyle et R^{b} = H et R¹ = Et (produit 5)

### Etape 1 : (idem produit 4)

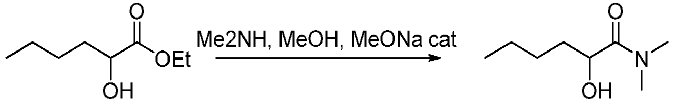

Dans un tricol, sont mélangés à 20°C et sous atmosphère inerte le 2-hydroxycaproate d'éthyle (24,24 g; 150 mmole) et le méthylate de sodium en solution dans le méthanol (25% p/p) (1,6 g; 7 mmole). A ce mélange est additionnée la diméthylamine en solution dans le méthanol (50% p/p) (26,75 g ; 297 mmole) en 30 minutes. La température de ce mélange est portée et maintenue pendant 3 heures à 40°C. Après retour à 20°C de la température de ce mélange réactionnel, de l'acide orthophosphorique est additionné jusqu'à obtenir un pH de 5. Les sels formés sont retirés par filtration et les solvants sont retirés sous pression réduite. Le 2-hydroxy-N,N-diméthylhexanamide est alors obtenu une pureté de 98% soit un rendement de 81%.

### Etape 2 :

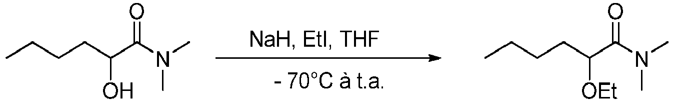

Dans un tricol, muni d'une ampoule à addition isobare, d'une entrée d'azote et de deux septa est introduit l'hydrure de sodium (60% en suspension dans l'huile minérale) (1,09 g, 27 mmole). Ce produit est lavé par du pentane (3 x 15 mL) et puis mis en suspension dans le THF (140 g). La suspension est refroidie à -70°C. Le 2-hydroxy-N,N-diméthylhexanamide (4,36 g, 30 mmole) est introduit avec une seringue. L'iodoéthane (6,40 g, 41 mmole) est alors additionné lentement sur le mélange. La température du mélange réactionnel est ramenée à l'ambiante et le mélange réactionnel est maintenu sous agitation pendant 20 heures. Le mélange réactionnel est neutralisé avec de l'eau froide (20 mL) puis cette phase aqueuse est extraite par de l'éther diéthylique (200 mL). La phase organique est lavée avec une solution saturée de NH₄Cl (50 mL), une solution saturée de NaHCO₃ (50 mL) puis de l'eau (50 mL). La phase organique est séchée sur sulfate de sodium et concentrée sous pression réduite pour donner 3,4 g du produit attendu avec une pureté > 98% soit un rendement de 52%.

### Exemple 7.3. - R^{a}= n-propyle et R^{b} = H et R¹ = Me (produit 6)

### Etape 1 :

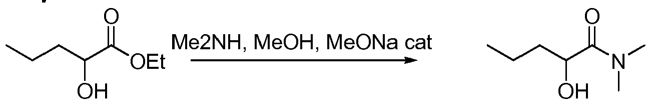

Dans un tricol, sont mélangés à 20°C et sous atmosphère inerte le 2-hydroxyvalérate d'éthyle (31 g ; 210 mmole) et le méthylate de sodium en solution dans le méthanol (25% p/p) (2,2 g ; 10 mmole). A ce mélange est additionnée la diméthylamine en solution dans le méthanol (50% p/p) (37,1 g ; 411 mmole) en 30 minutes. La température de ce mélange est portée et maintenue pendant 3 heures à 40°C. Après retour à 20°C de la température de ce mélange réactionnel, de l'acide orthophosphorique est additionné jusqu'à obtenir un pH de 5. Les sels formés sont retirés par filtration et les solvants sont retirés sous pression réduite. Le 2-hydroxy-N,N-diméthylhexanamide est alors obtenu une pureté < 98% soit un rendement de 80%.

### Etape 2 :

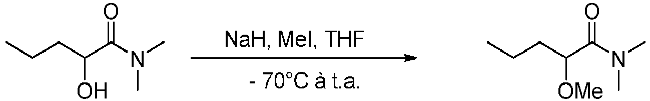

Dans un tricol, muni d'une ampoule à addition isobare, d'une entrée d'azote et de deux septa est introduit l'hydrure de sodium (60% en suspension dans l'huile minérale) (1,76 g, 44 mmole). Ce produit est lavé par du pentane (3 x 15 mL) et puis mis en suspension dans le THF. La suspension est refroidie à -70°C. Le 2-hydroxy-N,N-diméthylpentanamide (5,43 g, 40 mmole) est introduit avec une seringue. L'iodométhane (7,88 g, 55 mmole) est alors additionné lentement sur le mélange. La température du mélange réactionnel est ramenée à l'ambiante et le mélange réactionnel est maintenu sous agitation pendant 20 heures. Le mélange réactionnel est neutralisé avec de l'eau froide (20 mL) puis cette phase aqueuse est extraite par de l'éther diéthylique (200 mL). La phase organique est lavée avec une solution saturée de NH₄Cl (50 mL), une solution saturée de NaHCO₃ (50 mL) puis de l'eau (50 mL). La phase organique est séchée sur sulfate de sodium et concentrée sous pression réduite pour donner 2 g du produit attendu avec une pureté > 98% soit un rendement de 36%.

### Exemple 7.4. - R^{a}= n-propyle et R^{b} = H et R¹ = Et (produit 7)

### Etape 1 : (idem produit 6)

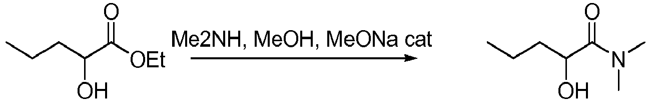

Dans un tricol, sont mélangés à 20°C et sous atmosphère inerte le 2-hydroxyvalérate d'éthyle (31 g ; 210 mmole) et le méthylate de sodium en solution dans le méthanol (25% p/p) (2,2 g ; 10 mmole). A ce mélange est additionnée la diméthylamine en solution dans le méthanol (50% p/p) (37,1 g ; 411 mmole) en 30 minutes. La température de ce mélange est portée et maintenue pendant 3 heures à 40°C. Après retour à 20°C de la température de ce mélange réactionnel, de l'acide orthophosphorique est additionné jusqu'à obtenir un pH de 5. Les sels formés sont retirés par filtration et les solvants sont retirés sous pression réduite. Le 2-hydroxy-N,N-diméthylhexanamide est alors obtenu une pureté < 98% soit un rendement de 80%.

### Etape 2 :

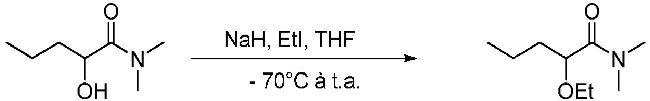

Dans un tricol, muni d'une ampoule à addition isobare, d'une entrée d'azote et de deux septa est introduit l'hydrure de sodium (60% en suspension dans l'huile minérale) (1,46 g, 37 mmole). Ce produit est lavé par du pentane (3 x 15 mL) et puis mis en suspension dans le THF (184g). La suspension est refroidie à -70°C. Le 2-hydroxy-N,N-diméthylpentanamide (5,31 g, 40 mmole) est introduit avec une seringue. L'iodoéthane (8,56 g, 55 mmole) est alors additionné lentement sur le mélange. La température du mélange réactionnel est ramenée à l'ambiante et le mélange réactionnel est maintenu sous agitation pendant 20 heures. Le mélange réactionnel est neutralisé avec de l'eau froide (20 mL) puis cette phase aqueuse est extraite par de l'éther diéthylique (200 mL). La phase organique est lavée avec une solution saturée de NH₄Cl (50 mL), une solution saturée de NaHCO₃ (50 mL) puis de l'eau (50 mL). La phase organique est séchée sur sulfate de sodium et concentrée sous pression réduite pour donner 4 g du produit attendu avec une pureté > 98% soit un rendement de 67%.

| Molécule | Solubilité dans l'eau (%p/p) |
|---|---|
| Produit 4 | <0,05 |
| Produit 5 | <0,05 |
| Produit 6 | <0,1 |
| Produit 7 | <0,1 |

## Revendications

1. Utilisation comme solvant, co-solvant, inhibiteur de cristallisation, ou agent plastifiant d'un composé de formule (I) suivante :
R^{a}R^{b}C(OR¹)-CONR²R³ (I)
où
- R^{a} est un groupe alkyle, linéaire ou ramifié, en C₁-C₆,
- R^{b} est H,
- R¹ est choisi parmi les groupes méthyle, éthyle, propyle, isopropyle, n-butyle, isobutyle, n-pentyle, isopentyle, isoamyle, n-hexyle, cyclohexyle, n-octyle, isooctyle, 2-éthylhexyle, décyle, dodécyle, tridécyle, phényle et 1-phényléthyle,
- R² et R³, identiques ou différents, sont des groupes hydrocarbonés comprenant un nombre moyen d'atomes de carbone allant de 1 à 15, éventuellement substitués, choisi parmi les groupes aliphatiques acycliques, saturés ou insaturés, linéaires ou ramifiés, les groupes carbocycliques saturés, insaturés ou aromatiques, monocycliques ou polycycliques et les enchaînements desdits groupes,
R² et R³ pouvant éventuellement former ensemble un cycle comprenant l'atome d'azote auquel ils sont liés, éventuellement substitué.

2. Utilisation selon la revendication 1, **caractérisée en ce que** le nombre total d'atomes de carbone, hors groupes R¹, R² et R³ est de 2, 3, 4, 5, 6, 7 ou 8.

3. Utilisation selon la revendication 1, **caractérisée en ce que** :
- R^{a} = Me ou Et, et
- R^{b} = H.

4. Utilisation selon l'une des revendications précédentes, **caractérisée en ce que** R² et R³, identiques ou différents, sont choisis parmi les groupes méthyle, éthyle, n-propyle, isopropyle, n-butyle, isobutyle, n-pentyle, amyle, isoamyle, hexyle, cyclohexyle, ou **en ce qu'**ils forment ensemble avec l'atome d'azote un groupe morpholine, pyrrolidine, pipérazine ou pipéridine.

5. Utilisation selon l'une quelconque des revendications 1 à 4, dans une formulation phytosanitaire, dans une formulation du nettoyage, dans une formulation de décapage, dans une formulation de dégraissage, dans une formulation de lubrifiants, dans une formulation de revêtement, dans une formulation de pigments ou encre, dans une formulation plastique.

6. Composé choisi parmi les composés suivants :

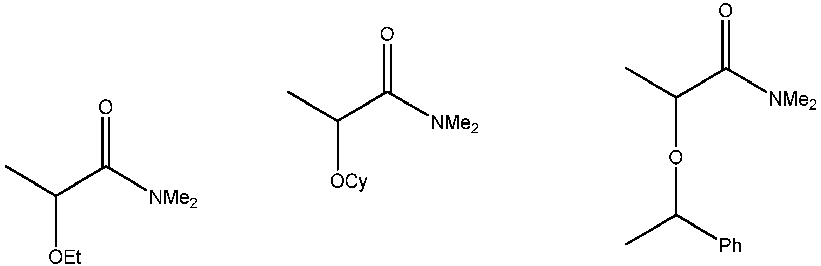

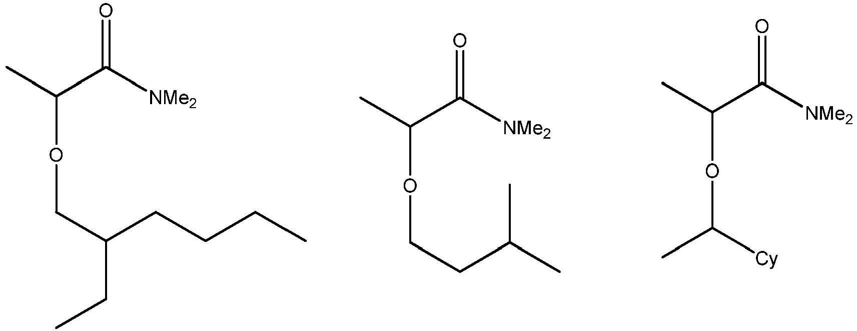

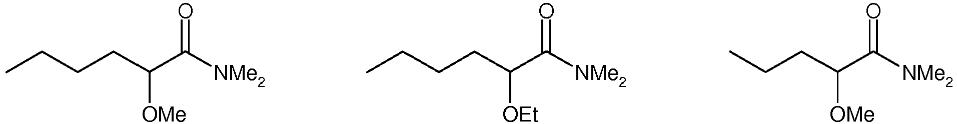

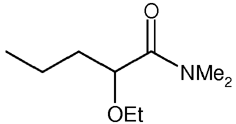

## Patentansprüche

1. Verwendung einer Verbindung der folgenden Formel (I)
R^{a}R^{b}C (OR¹) -CONR²R³ (I)
wobei
- R^{a} für eine lineare oder verzweigte C₁-C₆-Alkylgruppe steht,
- R^{b} für H steht,
- R¹ aus Methyl-, Ethyl-, Propyl-, Isopropyl-, n-Butyl-, Isobutyl-, n-Pentyl-, Isopentyl-, Isoamyl-, n-Hexyl-, Cyclohexyl-, n-Octyl-, Isooctyl-, 2-Ethylhexyl-, Decyl-, Dodecyl-, Tridecyl-, Phenyl- und 1-Phenylethylgruppen ausgewählt ist,
- R² und R³ gleich oder verschieden sind und für gegebenenfalls substituierte Kohlenwasserstoffgruppen mit einer durchschnittlichen Zahl von Kohlenstoffatomen im Bereich von 1 bis 15 stehen, die aus linearen oder verzweigten, gesättigten oder ungesättigten acyclischen aliphatischen Gruppen, monocyclischen oder polycyclischen gesättigten, ungesättigten oder aromatischen carbocyclischen Gruppen und Verknüpfungen dieser Gruppen ausgewählt sind,
wobei R² und R³ gegebenenfalls zusammen einen gegebenenfalls substituierten Ring, der das Stickstoffatom, an das sie gebunden sind, enthält, bilden können,
als Lösungsmittel, Cosolvens, Kristallisationsinhibitor oder Weichmacher.

2. Verwendung nach Anspruch 1, **dadurch gekennzeichnet, dass** die Gesamtzahl von Kohlenstoffatomen ohne die Gruppen R¹, R² und R³ 2, 3, 4, 5, 6, 7 oder 8 beträgt.

3. Verwendung nach Anspruch 1, **dadurch gekennzeichnet, dass**:
- R^{a}= Me oder Et und
- R^{b} = H.

4. Verwendung nach einem der vorhergehenden Ansprüche, **dadurch gekennzeichnet, dass** R² und R³ gleich oder verschieden sind und aus Methyl-, Ethyl-, n-Propyl-, Isopropyl-, n-Butyl-, Isobutyl-, n-Pentyl-, Amyl-, Isoamyl-, Hexyl- und Cyclohexylgruppen ausgewählt sind oder dass sie zusammen mit dem Stickstoffatom eine Morpholin-, Pyrrolidin-, Piperazin- oder Piperidingruppe bilden.

5. Verwendung nach einem der Ansprüche 1 bis 4 in einer Pflanzenschutzformulierung, in einer Reinigungsformulierung, in einer Beizformulierung, in einer Entfettungsformulierung, in einer Schmiermittelformulierung, in einer Beschichtungsformulierung, in einer Pigment- oder Tintenformulierung oder in einer Kunststoffformulierung.

6. Verbindung, ausgewählt aus den folgenden Verbindungen:

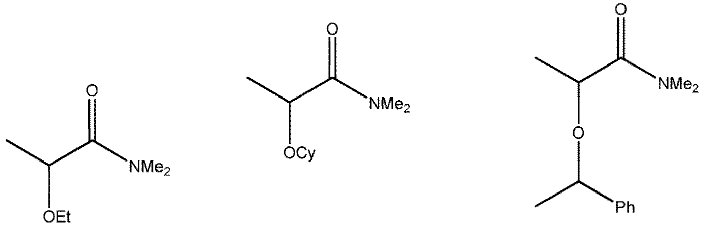

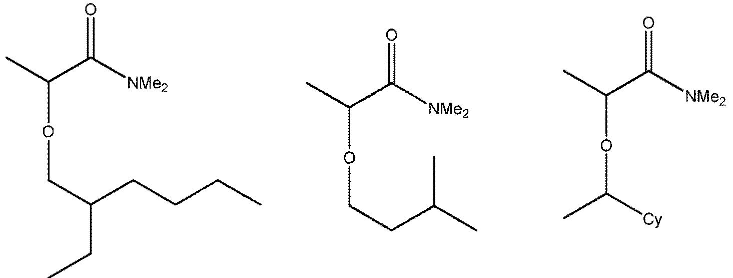

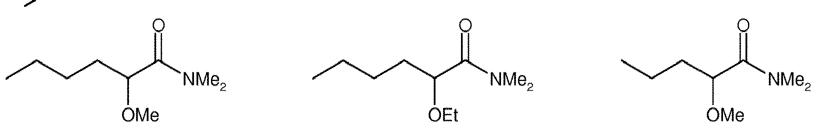

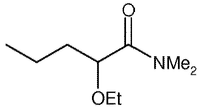

## Claims

1. Use as solvent, cosolvent, crystallization inhibitor or plasticizing agent of a compound of the following formula (I):
R^{a}R^{b}C(OR¹) -CONR²R³ (I)
where:
- R^{a} is a linear branched C₁-C₆ alkyl group,
- R^{b} is H,
- R¹ is chosen from the methyl, ethyl, propyl, isopropyl, n-butyl, isobutyl, n-pentyl, isopentyl, isoamyl, n-hexyl, cyclohexyl, n-octyl, isooctyl, 2-ethylhexyl, decyl, dodecyl, tridecyl, phenyl and 1-phenylethyl groups,
- R² and R³, which are identical or different, are optionally substituted hydrocarbon groups comprising a mean number of carbon atoms ranging from 1 to 15 chosen from saturated or unsaturated and linear or branched acyclic aliphatic groups, saturated, unsaturated or aromatic, monocyclic or polycyclic, carbocyclic groups and the sequences of the said groups,
it being possible for R² and R³ to optionally together form a ring comprising the nitrogen atom to which they are bonded, which is optionally substituted.

2. Use according to Claim 1, **characterized in that** the total number of carbon atoms, except for R¹, R² and R³ groups, is 2, 3, 4, 5, 6, 7, or 8.

3. Use according to Claim 1, **characterized in that**:
- R^{a} = Me or Et, and
- R^{b} = H.

4. Use according to one of the preceding claims, **characterized in that** R² and R³, which are identical or different, are chosen from the methyl, ethyl, n-propyl, isopropyl, n-butyl, isobutyl, n-pentyl, amyl, isoamyl, hexyl or cyclohexyl groups or **in that** they form, together with the nitrogen atom, a morpholine, pyrrolidine, piperazine or piperidine group.

5. Use according to any one of Claims 1 to 4, in a plant protection formulation, in a cleaning formulation, in a stripping formulation, in a degreasing formulation, in a lubricating formulation, in a coating formulation, in a pigment or ink formulation or in a plastic formulation.

6. Compound chosen from the following compounds:
